# EUROPEAN PATENT APPLICATION

(11) **EP 2 266 602 A2**
(43) Date of publication of application: **29.12.2010**
(21) Application number: 10178110.2
(22) Date of filing: 01.11.2005
(51) Int. Cl.: A61K 39/21, C07K 14/16

(54) **Combination approaches for generating immune responses**

(30) Priority: 01.11.2004 US 624506 P
(62) Divisional of application: 05816142.3
(71) Applicant: Novartis Vaccines and Diagnostics, Inc., Emeryville, CA 94608-2916 (US); The Government of the U.S.A. as represented by The Secretary of the dept. of Health & Human Services, Rockville, MD 20852-3804 (US)
(72) Inventor: Barnett, Susan W., Emeryville, CA 94662-8097 (US); Gomez-Roman, Victor Raul, Rockville, MD 20852-3804 (US); Robert-Guroff, Marjorie, Rockville, MD 20852-3804 (US); Srivastava, Indresh K., Emeryville, CA 94662-8097 (US)
(74) Representative: Hallybone, Huw George

(57) **Abstract**

The present invention relates to methods, polypeptides, and polynucleotides encoding immunogenic identical or analogous HIV polypeptides derived from the same or different strains within an HIV subtype and/or different subtypes. Uses of the polynucleotides and polypeptides in combination approaches for generating immune responses are also described. The combination approaches described herein induce broad and potent immune responses against diverse HIV strains from multiple strains within a given subtype and against diverse subtypes. Formulations of compositions for generating immune responses and methods of use for such compositions are also disclosed.

## Description

### TECHNICAL FIELD

The present invention relates to compositions comprising polynucleotide components and optionally a polypeptide component that can be used for the generation of immune responses in a subject. In one aspect, the compositions of the present invention are used in methods to generate immune responses in subjects to which the compositions are administered. In another aspect, the compositions of the present invention are used in methods of generating broad immune responses against multiple strains derived from a single subtype or serotype or multiple subtypes or serotypes of a selected microorganism, for example, Human Immunodeficiency Virus (HIV)).

### BACKGROUND

Acquired immune deficiency syndrome (AIDS) is recognized as one of the greatest health threats facing modem medicine. There is, as yet, no cure for this disease.

In 1983-1984, three groups independently identified the suspected etiological agent of AIDS. See, e.g., Barre-Sinoussi et al. (1983) Science 220:868-871; Montagnier et al., in Human T-Cell Leukemia Viruses (Gallo, Essex & Gross, eds., 1984); Vilmer et al. (1984) The Lancet 1:753; Popovic et al. (1984) Science 224:497-500; Levy et al. (1984) Science 225:840-842. These isolates were variously called lymphadenopathy-associated virus (LAV), human T-cell lymphotropic virus type III (HTLV-III), or AIDS-associated retrovirus (ARV). All of these isolates are strains of the same virus, and were later collectively named Human Immunodeficiency Virus (HIV). With the isolation of a related AIDS-causing virus, the strains originally called HIV are now termed HIV-1 and the related virus is called HIV-2 See, e.g., Guyader et al. (1987) Nature 326:662-669; Brun-Vezinet et al. (1986) Science 233:343-346; Clavel et al. (1986) Nature 324:691-695.

A great deal of information has been gathered about the HIV virus; however, to date an effective vaccine has not been identified. Several targets for vaccine development have been examined including the *env* and *Gag* gene products encoded by HIV. Gag gene products include, but are not limited to, Gag-polymerase and Gag-protease. Env gene products include, but are not limited to, monomeric gp120 polypeptides, oligomeric gp140 polypeptides and gp160 polypeptides.

Haas, et al., (Current Biology 6(3):315-324, 1996) suggested that selective codon usage by HIV-1 appeared to account for a substantial fraction of the inefficiency of viral protein synthesis. Andre, et al., (J. Virol. 72(2):1497-1503, 1998) described an increased immune response elicited by DNA vaccination employing a synthetic gp120 sequence with modified codon usage. Schneider, et al., (J Virol. 71(7):4892-4903, 1997) discuss inactivation of inhibitory (or instability) elements (INS) located within the coding sequences of the Gag and Gag-protease coding sequences.

The *Gag* proteins of HIV-1 are necessary for the assembly of virus-like particles. HIV-1 *Gag* proteins are involved in many stages of the life cycle of the virus including, assembly, virion maturation after particle release, and early post-entry steps in virus replication. The roles of HIV-1 *Gag* proteins are numerous and complex (Freed, E.O., Virology 251:1-15, 1998).

Wolf, et al., (PCT International Publication No. WO 96/30523, published 3 October 1996; European Patent Application, Publication No. 0 449 116 A1, published 2 October 1991) have described the use of altered pr55 *Gag* of HIV-1 to act as a non-infectious retroviral-like particulate carrier, in particular, for the presentation of immunologically important epitopes. Wang, et al., (Virology 200:524-534, 1994) describe a system to study assembly of HIV Gag-beta-galactosidase fusion proteins into virions. They describe the construction of sequences encoding HIV Gag-beta-galactosidase fusion proteins, the expression of such sequences in the presence of HIV Gag proteins, and assembly of these proteins into virus particles.

Shiver, et al., (PCT International Publication No. WO 98/34640, published 13 August 1998) described altering HIV-1 (CAM1) *Gag* coding sequences to produce synthetic DNA molecules encoding HIV *Gag* and modifications of HIV *Gag*. The codons of the synthetic molecules were codons preferred by a projected host cell.

Recently, use of HIV Env polypeptides in immunogenic compositions has been described. (see, U.S. Patent No. 5,846,546 to Hurwitz et al., issued December 8, 1998, describing immunogenic compositions comprising a mixture of at least four different recombinant virus that each express a different HIV env variant; and U.S. Patent No. 5,840,313 to Vahlne et al., issued November 24, 1998, describing peptides which correspond to epitopes of the HIV-1 gp 120 protein). In addition, U.S. Patent No. 5,876,731 to Sia et al, issued March 2, 1999 describes candidate vaccines against HIV comprising an amino acid sequence of a T-cell epitope of Gag linked directly to an amino acid sequence of a B-cell epitope of the V3 loop protein of an HIV-1 isolate containing the sequence GPGR.

PCT International Publication Nos. WO/00/39302; WO/00/39303; WO/00/39304; WO/02/04493; WO/03/004657; WO/03/004620; and WO/03/020876 described a number of codon-optimized HIV polypeptides, as well as some native HIV sequences. Further, a variety of HIV polypeptides comprising mutations were described. The use of these HIV polypeptides in vaccine compositions and methods of immunization were also described.

The present invention provides improved compositions and methods for generating immune responses against multiple subtypes, serotypes, or strains of a selected microorganism, for example, a virus (e.g., HIV-1).

### SUMMARY

The present invention relates to compositions and methods for their use for generating an immune response in a subject. The compositions of the invention comprise at least two components wherein each component comprises an identical or analogous polypeptide immunogen. The polypeptide immunogen is provided either directly in the form of a polypeptide (including polypeptide fragments, modified forms, encapsulated forms, etc.) or in a preferred embodiment indirectly as a polynucleotide immunogen (including DNA and/or RNA encoding a polypeptide immunogen) encoded in a gene delivery vector.

The compositions of the present invention may be used in methods to generate immune responses in subjects to which the compositions are administered, wherein the immune response is directed against multiple subtypes, serotypes, or strains of a selected microorganisms, for example, viruses (e.g., Human Immunodeficiency Virus (HIV)). In a preferred embodiment, the present invention relates to compositions comprising two or more different polynucleotide components (e.g., a replicating or non-replicating adenovirus vector in combination with a nonreplicating alphavirus vector) encoding an identical or analogous polypeptide and one or more optional polypeptide components that can be used for the generation of immune responses in a subj ect, for example, the generation of neutralizing antibodies, ADCC activity and T-cell responses.

The compositions of the present invention may be used in methods to generate immune responses in subjects to which the compositions are administered, wherein the immune response is directed against multiple strains of a first subtype or serotype or against multiple subtypes or serotypes of a selected microorganims, for example, viruses (e.g., Human Immunodeficiency Virus (HIV)). In another embodiment, the immunogens may each be delivered with a viral vector, preferably different vectors. For example, a first polypeptide as immunogen may be encoded in a polynucleotide that is delivered to a subject by way of an adenoviral vector or an alphavirus vector. Subsequently or simultaneously, a second identical or analogous polypeptide as immunogen may be delivered by way of another adenovirus or an alphavirus vector. The first and second identical or analogous immunogens can be from the same or different HIV strains of the same subtype or different HIV subtypes.

In other aspects, the compositions further comprise a polypeptide component comprising one or more HIV immunogenic polypeptides identical or analogous to the polypeptide encoded by the polynucleotide components. The polypeptide(s) may be derived from the same strains or subtypes as one or more of the polynucleotide components or may be derived from yet a different strains or subtypes.

The first and second (priming and boosting) gene delivery vectors described herein may comprise at least one polynucleotide that is a native polynucleotide. Alternately, or in addition, the priming and boosting gene delivery vectors may comprise at least one polynucleotide that is a synthetic polynucleotide. Synthetic polynucleotides may comprise codons optimized for expression in mammalian cells (e.g., human cells). The gene delivery vectors may comprise a single polynucleotide molecule, or two or more different polynucleotide molecules, each encoding one or more HIV polypeptides. The gene delivery vectors may comprise DNA or RNA or both.

The optional HIV immunogenic polypeptides (encoded by the polynucleotide component and/or those which comprise the polypeptide component) may be HIV envelope, Gag or other HIV polypeptides. The gene delivery vectors made encode HIV polypeptides that comprise one or more mutations compared to the wild-type (i.e., naturally-occurring) HIV polypeptide (e.g., in the case of envelope proteins, at least one of the envelope polypeptides may comprise a mutation in the cleavage site or a mutation in the glycosylation site, a deletion or modification of the V1 region, a deletion or modification of the V2 region, a deletion or modification of the V3 region, modifications to expose an envelope binding region that binds to a CCR5 chemokine co-receptor, and combinations thereof). Mutations in the envelope protein may also expose antibody binding sites to other receptors that are involved in viral binding and/or entry. Furthermore, other immunogenic HIV polypeptides may include, but are not limited to, Gag, Env, Pol, Prot, Int, RT, vif, vpr, vpu, tat, rev, and nef polypeptides.

The first subtype from which the HIV immunogenic polypeptides and coding sequences therefore may be selected includes, but are not limited to, the following: subtypeA, subtypeB, subtypeC, subtypeD, subtypeE, subtypeF, subtypeG, and subtype O, as well as any of the identified CRFs.

In addition to immunogenic HIV polypeptides and sequences encoding same, the gene delivery vectors may encode and the optional polypeptide component may comprise one or more additional antigenic polypeptides that may include antigenic polypeptides not derived from HIV-1 coding sequences.

One or more of the gene delivery vectors may further comprise sequences encoding one or more control elements compatible with expression in a selected host cell, wherein the control elements are operable linked to polynucleotides encoding HIV immunogenic polypeptides. Exemplary control elements include, but are not limited to, a transcription promoter (e.g., CMV, CMV+intron A, SV40, RSV, HIV-Ltr, MMLV-1tr, and metallothionein), a transcription enhancer element, a transcription termination signal, polyadenylation sequences, sequences for optimization of initiation of translation, internal ribosome entry sites, and translation termination sequences.

The gene delivery vector(s) may comprise further components as described herein (e.g., carriers, control sequences, etc.). The polypeptide component may comprise further components as described herein (e.g., carriers, adjuvants, immunoenhancers, etc.).

The present invention also includes methods of generating an immune response in a subject, for example by administering any of the compositions described herein to the subject. In certain embodiments, the methods comprise administering a composition comprising a first gene delivery vector (also referred to as a priming vector), the first gene delivery vector comprising the polynucleotides of a first polynucleotide component encoding a first HIV immunogenic polypeptide are administered to the subject under conditions that are compatible with expression of the polynucleotides in the subject for the production of encoded HIV immunogenic polypeptides. Concurrently or subsequently, a composition comprising a second gene delivery vector (also referred to as a boosting vector) is administered to the subject. The first and second gene delivery vectors can be, for example, replicating or non-replicating adenovirus vectors or alphavirus vectors (e.g., nonreplicating).

In yet other aspects, the methods of generating an immune response further comprise administering one or more polypeptide components as described herein. The first and second gene delivery vectors and the polypeptide component may be administered, for example, concurrently or sequentially. The optional polypeptide component may comprise further components as described herein (e.g., carriers, adjuvants, immunoenhancers, etc.) and may be soluble or particulate.

The one or more gene delivery vectors may comprise, for example, nonviral and/or viral vectors. Exemplary viral vectors include, but are not limited to retroviral, lentiviral, alphaviral, poxviral, herpes viral, adeno-associated viral, polioviral, measles viral, adenoviral vectors, or other known viral vectors. In a preferred embodiment, the first and second gene delivery vectors are alphavirus or adenovirus vectors. In particularly preferred embodiments, the second (boosting) gene delivery vector is a nonreplicating adenovirus vector or a nonreplicating alphavirus vector.

The gene delivery vectors may be delivered using a particulate carrier, for example, coated on a gold or tungsten particle and the coated particle may be delivered to the subject using a gene gun, or PLG particles delivered by electroporation or otherwise. Alternatively, the gene delivery vectors may be encapsulated in a liposome preparation.

The gene delivery vectors and/or polypeptides may be administered, for example, intramuscularly, intramucosally, intranasally, subcutaneously, intradermally, transdermally, intravaginally, intrarectally, orally, intravenously, or by combinations of these methods.

The subjects of the methods of the present invention are typically mammals, for example, humans.

The immune response generated by the methods of the present invention may be humoral and/or cellular. In one embodiment, the immune response results in generating broadly neutralizing antibodies in the subject against multiple strains derived from the first HIV subtype or against multiple subtypes. In another embodiment, the immune response results in broadly neutralizing antibodies against multiple strains derived from different subtypes.

These and other embodiments of the present invention will readily occur to those of ordinary skill in the art in view of the disclosure herein.

### DETAILED DESCRIPTION

The practice of the present invention will employ, unless otherwise indicated, conventional methods of chemistry, biochemistry, molecular biology, immunology and pharmacology, within the skill of the art. Such techniques are explained fully in the literature. See, e.g., Remington's Pharmaceutical Sciences, 18th Edition (Easton, Pennsylvania: Mack Publishing Company, 1990); Methods In Enzymology (S. Colowick and N. Kaplan, eds., Academic Press, Inc.); and Handbook of Experimental Immunology, Vols. I-IV (D.M. Weir and C.C. Blackwell, eds., 1986, Blackwell Scientific Publications); Sambrook, et al., Molecular Cloning: A Laboratory Manual (2nd Edition, 1989); Short Protocols in Molecular Biology, 4th ed. (Ausubel et al. eds., 1999, John Wiley & Sons); Molecular Biology Techniques: An Intensive Laboratory Course, (Ream et al., eds., 1998, Academic Press); PCR (Introduction to Biotechniques Series), 2nd ed. (Newton & Graham eds., 1997, Springer Verlag).

All patents, publications, sequence citations, and patent applications cited in this specification are herein incorporated by reference as if each individual patent, publication, sequence citation, or patent application was specifically and individually indicated to be incorporated by reference in its entirety for all purposes.

As used in this specification, the singular forms "a," "an" and "the" include plural references unless the content clearly dictates otherwise. Thus, for example, reference to "an antigen" includes a mixture of two or more such agents.

### 1.0.0 DEFINITIONS

In describing the present invention, the following terms will be employed, and are intended to be defined as indicated below.

"Synthetic" sequences, as used herein, refers to HIV polypeptide-encoding polynucleotides whose expression has been modified as described herein, for example, by codon substitution, altered activities, and/or inactivation of inhibitory sequences. "Wild-type" or "native" sequences, as used herein, refer to polypeptide-encoding polynucleotides that are substantially as they are found in nature, e.g., Gag, Pol, Vif, Vpr, Tat, Rev, Vpu, Env and/or Nef encoding sequences as found in HIV isolates, e.g., SF162, SF2, AF110965, AF110967, AF110968, AF110975, MJ4 (a subtype C, Ndung'u et al. (2001) J. Virol. 75:4964-4972), subtype B-SF162, subtype C-TV1.8_2 (8_2_TV1_C.ZA), subtype C-TV1.8_5 (8_5_TV1_C.ZA), subtype C-TV2.12-5/1 (12-5_1_TV2_C.ZA), subtype C-MJ4, India subtype C-93IN101, subtype A-Q2317, subtype D-92UG001, subtype E-cm235, subtype A HIV-1 isolate Q23-17 from Kenya GenBank Accession AF004885, subtype A HIV-1 isolate 98UA0116 from Ukraine GanBank Accession AF413987, subtype A HIV-1 isolate SE8538 from Tanzania GenBank Accession AF069669, subtype A Human immunodeficiency virus 1 proviral DNA, complete genome, clone:pUG031-A1 GenBank Accession AB098330, subtype D Human immunodeficiency virus type 1 complete proviral genome, strain 92UG001 GenBank Accession AJ320484, subtype D HIV-1 isolate 94UG114 from Uganda GenBank Accession U88824, subtype D Human immunodeficiency virus type 1, isolate ELIGenBank Accession K03454, and Indian subtype C Human immunodeficiency virus type 1 subtype C genomic RNA GenBank Accession AB023804.

The various regions of the HIV genome are shown in Table 1, with numbering relative to 8_5_TV1_C.ZA. Thus, the term "Pol" refers to one or more of the following polypeptides: polymerase (p6Pol); protease (prot); reverse transcriptase (p66RT or RT); RNAseH (p15RNAseH); and/or integrase (p31Int or Int). Identification of gene regions for any selected HIV isolate (e.g., strains within a subtype, or strains derived from different subtypes) can be performed by one of ordinary skill in the art based on the teachings presented herein and the information known in the art, for example, by performing nucleotide and/or polypeptide alignments relative to 8_5_TV1_C.ZA or alignment to other known HIV isolates, for example, Subtype B isolates with gene regions (e.g., SF2, GenBank Accession number K02007; SF162, GenBank Accession Number M38428) and Subtype C isolates with gene regions (e.g., GenBank Accession Number AF110965 and GenBank Accession Number AF110975).

HIV-1 is classified by phylogenetic analysis into three groups: group M (major), group 0 (outlier) and a variant of HIV-1, designated group N. Subtypes (clades) represent different lineages of HIV and have geographic associations. Subtypes of HIV-1 are phylogenetically associated groups of HIV-1 sequences, with the sequences within any one subtype or sub-subtype more similar to each other than to sequences from different subtypes throughout their genomes. See, e.g., Los Alamos National Laboratory HIV Sequence Database (http://hiv-web.lanl.gov/content/hiv-db/HelpDocs/subtypes-more.html) (Los Alamos, NM).

The HIV-1 M group subtypes are phylogenetically associated groups or clades of HIV-1 sequences, and include subtypes A (e.g., A1, A2), B, C, D, F (e.g., F1, F2), G, H, J and K. Subtypes and sub-subtypes of the HIV-1 M group are thought to have diverged in humans, following a single chimpanzee-to-human transmission event. The worldwide distribution of various HIV-1 M group subtypes is diverse, with subtype B being most prevalent in North America and Europe and subtype A being most prevalent in Africa. Whereas most subtypes are common in Central Africa, other areas have restricted distribution of genotypes. For example, subtype C is common in India and South Africa, and subtype F is prevalent in Romania, Brazil and Argentina. The HIV-1 M group also includes circulating recombinant forms (CRFs), which are viruses whose complete genome is a recombinant or mosaic consisting of some regions which cluster with one subtype and other regions of the genome which cluster with another subtype in phylogenetic analyses. Examples of CRFs are found in the Los Alamos National Laboratory HIV Sequence Database (http://www.hiv.lanl.gov/content/hiv-db/mainpage.html) (Los Alamos, NM). CRFs have also been referred to in the art as subtypes E and I. CRFs (subtype E) are highly prevalent in Thailand.

As used herein, the term "virus-like particle" or "VLP" refers to a nonreplicating, viral shell, derived from any of several viruses discussed further below. VLPs are generally composed of one or more viral proteins, such as, but not limited to those proteins referred to as capsid, coat, shell, surface and/or envelope proteins, or particle-forming polypeptides derived from these proteins. VLPs can form spontaneously upon recombinant expression of the protein in an appropriate expression system. Methods for producing particular VLPs are known in the art and discussed more fully below. The presence of VLPs following recombinant expression of viral proteins can be detected using conventional techniques known in the art, such as by electron microscopy, X-ray crystallography, and the like. See, e.g., Baker et al., Biophys. J. (1991) 60:1445-1456; Hagensee et al., J. Virol. (1994) 68:4503-4505. For example, VLPs can be isolated by density gradient centrifugation and/or identified by characteristic density banding. Alternatively, cryoelectron microscopy can be performed on vitrified aqueous samples of the VLP preparation in question, and images recorded under appropriate exposure conditions.

By "particle-forming polypeptide" derived from a particular viral protein is meant a full-length or near full-length viral protein, as well as a fragment thereof, or a viral protein with internal deletions, which has the ability to form VLPs under conditions that favor VLP formation. Accordingly, the polypeptide may comprise the full-length sequence, fragments, truncated and partial sequences, as well as analogs and precursor forms of the reference molecule. The term therefore intends deletions, additions and substitutions to the sequence, so long as the polypeptide retains the ability to form a VLP. Thus, the term includes natural variations of the specified polypeptide since variations in coat proteins often occur between viral isolates. The term also includes deletions, additions and substitutions that do not naturally occur in the reference protein, so long as the protein retains the ability to form a VLP. Preferred substitutions are those which are conservative in nature, i.e., those substitutions that take place within a family of amino acids that are related in their side chains. Specifically, amino acids are generally divided into four families: (1) acidic -- aspartate and glutamate; (2) basic -- lysine, arginine, histidine; (3) non-polar -- alanine, valine, leucine, isoleucine, proline, phenylalanine, methionine, tryptophan; and (4) uncharged polar -- glycine, asparagine, glutamine, cystine, serine threonine, tyrosine. Phenylalanine, tryptophan, and tyrosine are sometimes classified as aromatic amino acids.

The term "HIV polypeptide" refers to any amino acid sequence that exhibits sequence homology to native HIV polypeptides (*e*.*g*., Gag, Env, Prot, Pol, RT, Int, vif, vpr, vpu, tat, rev, nef and/or combinations thereof) and/or which is functional. Non-limiting examples of functions that may be exhibited by HIV polypeptides include, use as immunogens (*e*.*g*., to generate a humoral and/or cellular immune response), use in diagnostics (*e*.*g*, bound by suitable antibodies for use in ELISAs or other immunoassays) and/or polypeptides which exhibit one or more biological activities associated with the wild type or synthetic HIV polypeptide. For example, as used herein, the term "Gag polypeptide" may refer to a polypeptide that is bound by one or more anti-Gag antibodies; elicits a humoral and/or cellular immune response; and/or exhibits the ability to form particles.

An "antigen" refers to a molecule containing one or more epitopes (either linear, conformational or both) that will stimulate a host's immune system to make a humoral and/or cellular antigen-specific response. The term is used interchangeably with the term "immunogen." Normally, a B-cell epitope will include at least about 5 amino acids but can be as small as 3-4 amino acids. A T-cell epitope, such as a CTL epitope, will include at least about 7-9 amino acids, and a helper T-cell epitope at least about 12-20 amino acids. Normally, an epitope will include between about 7 and 15 amino acids, such as, 9, 10, 12 or 15 amino acids. The term "antigen" denotes both subunit antigens, (i.e., antigens which are separate and discrete from a whole organism with which the antigen is associated in nature), as well as, killed, attenuated or inactivated bacteria, viruses, fungi, parasites or other microbes. Antibodies such as anti-idiotype antibodies, or fragments thereof, and synthetic peptide mimotopes, which can mimic an antigen or antigenic determinant, are also captured under the definition of antigen as used herein. Similarly, an oligonucleotide or polynucleotide which expresses an antigen or antigenic determinant *in vivo,* such as in gene therapy and DNA immunization applications, is also included in the definition of antigen herein. Furthermore, the oligonucleotide or polynucleotide which expresses the antigen or immunogen may be delivered by a viral vector.

For purposes of the present invention, antigens (e.g., polynucleotide encoding antigens, or polypeptides comprising antigens) can be derived from any microorganism having more than one subtype, serotype, or strain variation (e.g., viruses, bacteria, parasites, fungi, etc.). The term also intends any of the various tumor antigens. Furthermore, for purposes of the present invention, an "antigen" refers to a protein which includes modifications, such as deletions, additions and substitutions (generally conservative in nature), to the native sequence, so long as the protein maintains the ability to elicit an immunological response, as defined herein. These modifications may be deliberate, as through site-directed mutagenesis, or may be accidental, such as through mutations of hosts which produce the antigens.

"Identical" as used herein in the context of HIV immunogenic polypeptides is meant to encompass a protein from the same gene of the same HIV strain. The phrase in this context is also meant to include "identical" polypeptides wherein one or more of the identical polypeptides are modified as described herein. For example, identical env polypeptides are meant to include e.g., a mutated or modified env protein, a wildtype or unmodified env protein from the same strain, or a different modification of the same gene from the same strain. The modifications can be the same or different, so long as the starting gene is from the same strain.

An "immunological response" to an antigen or composition is the development in a subject of a humoral and/or a cellular immune response to an antigen present in the composition of interest. For purposes of the present invention, a "humoral immune response" refers to an immune response mediated by antibody molecules, while a "cellular immune response" is one mediated by T-lymphocytes and/or other white blood cells. One important aspect of cellular immunity involves an antigen-specific response by cytolytic T-cells ("CTL"s). CTLs have specificity for peptide antigens that are presented in association with proteins encoded by the major histocompatibility complex (MHC) and expressed on the surfaces of cells. CTLs help induce and promote the destruction of intracellular microbes, or the lysis of cells infected with such microbes. Another aspect of cellular immunity involves an antigen-specific response by helper T-cells. Helper T-cells act to help stimulate the function, and focus the activity of, nonspecific effector cells against cells displaying peptide antigens in association with MHC molecules on their surface. A "cellular immune response" also refers to the production of cytokines, chemokines and other such molecules produced by activated T-cells and/or other white blood cells, including those derived from CD4+ and CD8+ T-cells.

A composition or vaccine that elicits a cellular immune response may serve to sensitize a vertebrate subject by the presentation of antigen in association with MHC molecules at the cell surface. The cell-mediated immune response is directed at, or near, cells presenting antigen at their surface. In addition, antigen-specific T-lymphocytes can be generated to allow for the future protection of an immunized host.

The ability of a particular antigen to stimulate a cell-mediated immunological response may be determined by a number of assays, such as by lymphoproliferation (lymphocyte activation) assays, CTL cytotoxic cell assays, or by assaying for T-lymphocytes specific for the antigen in a sensitized subject. Such assays are well known in the art. See, e.g., Erickson et al., J. Immunol. (1993) 151:4189-4199; Doe et al., Eur. J. Immunol. (1994) 24:2369-2376. Recent methods of measuring cell-mediated immune response include measurement of intracellular cytokines or cytokine secretion by T-cell populations, or by measurement of epitope specific T-cells (e.g., by the tetramer technique)(reviewed by McMichael, A.J., and O'Callaghan, C.A., J. Exp. Med. 187(9)1367-1371,1998; Mcheyzer-Williams, M.G., et al, Immunol. Rev. 150:5-21, 1996; Lalvani, A., et al, J. Exp. Med. 186:859-865,1997).

Thus, an immunological response as used herein may be one that stimulates the production of antibodies (e.g., neutralizing antibodies that block bacterial toxins and pathogens such as viruses entering cells and replicating by binding to toxins and pathogens, typically protecting cells from infection and destruction). The antigen of interest may also elicit production of CTLs. Hence, an immunological response may include one or more of the following effects: the production of antibodies by B-cells; and/or the activation of suppressor T-cells and/or memory/effector T-cells directed specifically to an antigen or antigens present in the composition or vaccine of interest. These responses may serve to neutralize infectivity, and/or mediate antibody-complement, or antibody dependent cell cytotoxicity (ADCC) to provide protection to an immunized host. Such responses can be determined using standard immunoassays and neutralization assays, well known in the art. (See, *e*.*g*., Montefiori et al. (1988) J. Clin Microbiol. 26:231-235; Dreyer et al. (1999) AIDS Res Hum Retroviruses (1999) 15(17):1563-1571). The innate immune system of mammals also recognizes and responds to moleluclar features of pathogenic organisms via activation of Toll-like receptors and similar receptor molecules on immune cells. Upon activation of the innate immune system, various non-adaptive immune response cells. are activated to, *e*.*g*., produce various cytokines, lymphokines and chemokines. Cells activated by an innate immune response include immature and mature Dendritic cells of the moncyte and plamsacytoid lineage (MDC, PDC), as well as gamma, delta, alpha and beta T cells and B cells and the like. Thus, the present invention also contemplates an immune response wherein the immune response involves both an innate and adaptive response.

An "immunogenic HIV polypeptide" is a polypeptide capable of eliciting an immune response against one or more native HIV polypeptides, when the immunogenic polypeptide is administered to a laboratory test animal (such as a mouse, guinea pig, rhesus macaque, chimpanzee, baboon, etc.).

An "immunogenic composition" is a composition that comprises an antigenic molecule where administration of the composition to a subject results in the development in the subject of a humoral and/or a cellular immune response to the antigenic molecule of interest. The immunogenic composition can be introduced directly into a recipient subject, such as by injection, inhalation, oral, intranasal and mucosal (*e*.*g*., intra-rectally or intra-vaginally) administration.

The term "subtypes" includes the subtypes currently identified as well as circulating recombinant forms (CRFs). HIV subtypes (including CRFs) are continually being characterized and can be found on the HIV database from Los Alamos National Laboratories, available on the internet. Subtypes include subtypes A (e.g., A1, A2), B, C, D, F (e.g., F1, F2), G, H, J and K, as well as various CRFs).

By "epitope" is meant a site on an antigen to which specific B cells and/or T cells respond, rendering the molecule including such an epitope capable of eliciting an immunological reaction or capable of reacting with HIV antibodies present in a biological sample. The term is also used interchangeably with "antigenic determinant" or "antigenic determinant site." An epitope can comprise three (3) or more amino acids in a spatial conformation unique to the epitope. Generally, an epitope consists of at least five (5) such amino acids and, more usually, consists of at least 8-10 such amino acids. Methods of determining spatial conformation of amino acids are known in the art and include, for example, x-ray crystallography and two-dimensional nuclear magnetic resonance. Furthermore, the identification of epitopes in a given protein is readily accomplished using techniques well known in the art, such as by the use of hydrophobicity studies and by site-directed serology. See, also, Geysen et al. (1984) Proc. Natl. Acad. Sci. USA 81:3998-4002 (general method of rapidly synthesizing peptides to determine the location of immunogenic epitopes in a given antigen); U.S. Patent No. 4,708,871 (procedures for identifying and chemically synthesizing epitopes of antigens); and Geysen et al. (1986) Molecular Immunology 23:709-715 (technique for identifying peptides with high affinity for a given antibody). Antibodies that recognize the same epitope can be identified in a simple immunoassay showing the ability of one antibody to block the binding of another antibody to a target antigen.

By "subunit vaccine" is meant a vaccine composition which includes one or more selected antigens but not all antigens, derived from or homologous to, an antigen from a pathogen of interest such as from a virus, bacterium, parasite or fungus. Such a composition is substantially free of intact pathogen cells or pathogenic particles, or the lysate of such cells or particles. Thus, a "subunit vaccine" can be prepared from at least partially purified (preferably substantially purified) immunogenic polypeptides from the pathogen, or analogs thereof. The method of obtaining an antigen included in the subunit vaccine can thus include standard purification techniques, recombinant production, or synthetic production.

"Substantially purified" general refers to isolation of a substance (compound, polynucleotide, protein, polypeptide, polypeptide composition) such that the substance comprises the majority percent of the sample in which it resides. Typically in a sample a substantially purified component comprises 50%, preferably 80%-85%, more preferably 90-95% of the sample. Techniques for purifying polynucleotides and polypeptides of interest are well-known in the art and include, for example, ion-exchange chromatography, affinity chromatography and sedimentation according to density.

A "polynucleotide coding sequence" or a polynucleotide sequence that "encodes" a selected polypeptide, is a nucleic acid molecule that is transcribed (in the case of DNA) and translated (in the case of mRNA) into a polypeptide in vivo when placed under the control of appropriate regulatory sequences (or "control elements"). The boundaries of the coding sequence are determined by a start codon, for example, at or near the 5' terminus and a translation stop codon, for example, at or near the 3' terminus. A coding sequence can include, but is not limited to, cDNA from viral, procaryotic or eucaryotic mRNA, genomic DNA sequences from viral or procaryotic DNA, and even synthetic DNA sequences. Exemplary coding sequences are codon optimized viral polypeptide-coding sequences used in the present invention. The coding regions of the polynucleotide sequences of the present invention are identifiable by one of skill in the art and may, for example, be easily identified by performing translations of all three frames of the polynucleotide and identifying the frame corresponding to the encoded polypeptide, for example, a synthetic nef polynucleotide of the present invention encodes a nef-derived polypeptide. A transcription termination sequence may be located 3' to the coding sequence.

Typical "control elements", include, but are not limited to, transcription regulators, such as promoters, transcription enhancer elements, transcription termination signals, and polyadenylation sequences; and translation regulators, such as sequences for optimization of initiation of translation, e.g., Shine-Dalgarno (ribosome binding site) sequences, internal ribosome entry sites (IRES) such as the ECMV IRES, Kozak-type sequences (i.e., sequences for the optimization of translation, located, for example, 5' to the coding sequence, e.g., GCCACC placed in front (5') of an initiating ATG), leader sequences, translation initiation codon (e.g., ATG), and translation termination sequences (e.g., TAA or, preferably, TAAA placed after (3') the coding sequence). In certain embodiments, one or more translation regulation or initiation sequences (e.g., the leader sequence) are derived from wild-type translation initiation sequences, *i*.*e*., sequences that regulate translation of the coding region in their native state. Wildtype leader sequences that have been modified, using the methods described herein, also find use in the present invention. Promoters can include inducible promoters (where expression of a polynucleotide sequence operably linked to the promoter is induced by an analyte, cofactor, regulatory protein, etc.), repressible promoters (where expression of a polynucleotide sequence operably linked to the promoter is induced by an analyte, cofactor, regulatory protein, etc.), and constitutive promoters.

A "nucleic acid" molecule or "polynucleotide" can include, but is not limited to, procaryotic sequences, eucaryotic mRNA, cDNA from eucaryotic mRNA, genomic DNA sequences from eucaryotic (e.g., mammalian) DNA, and even synthetic DNA sequences. The term also captures sequences that include any of the known base analogs of DNA and RNA. In referring to the polynucleotide of the invention, in those examples in which "DNA" is specifically recited, it will be apparent that for many such embodiments, RNA is likewise intended.

"Operably linked" refers to an arrangement of elements wherein the components so described are configured so as to perform their usual function. Thus, a given promoter operably linked to a coding sequence is capable of effecting the expression of the coding sequence when the proper enzymes are present. The promoter need not be contiguous with the coding sequence, so long as it functions to direct the expression thereof. Thus, for example, intervening untranslated yet transcribed sequences can be present between the promoter sequence and the coding sequence and the promoter sequence can still be considered "operably linked" to the coding sequence.

"Recombinant" as used herein to describe a nucleic acid molecule means a polynucleotide of genomic, cDNA, semisynthetic, or synthetic origin which, by virtue of its origin or manipulation: (1) is not associated with all or a portion of the polynucleotide with which it is associated in nature; and/or (2) is linked to a polynucleotide other than that to which it is linked in nature. The term "recombinant" as used with respect to a protein or polypeptide means a polypeptide produced by expression of a recombinant polynucleotide. "Recombinant host cells,""host cells," "cells," "cell lines," "cell cultures," and other such terms denoting procaryotic microorganisms or eucaryotic cell lines cultured as unicellular entities, are used interchangeably, and refer to cells which can be, or have been, used as recipients for recombinant vectors or other transfer DNA, and include the progeny of the original cell which has been transfected. It is understood that the progeny of a single parental cell may not necessarily be completely identical in morphology or in genomic or total DNA complement to the original parent, due to accidental or deliberate mutation. Progeny of the parental cell which are sufficiently similar to the parent to be characterized by the relevant property, such as the presence of a nucleotide sequence encoding a desired peptide, are included in the progeny intended by this definition, and are covered by the above terms.

Techniques for determining amino acid sequence "similarity" are well known in the art. In general, "similarity" means the exact amino acid to amino acid comparison of two or more polypeptides at the appropriate place, where amino acids are identical or possess similar chemical and/or physical properties such as charge or hydrophobicity. A so-termed "percent similarity" then can be determined between the compared polypeptide sequences. Techniques for determining nucleic acid and amino acid sequence identity also are well known in the art and include determining the nucleotide sequence of the mRNA for the gene encoding the amino acid sequence (usually via a cDNA intermediate) and determining the amino acid sequence encoded thereby, and comparing this to a second amino acid sequence. In general, "identity" refers to an exact amino acid to amino acid or nucleotide to nucleotide correspondence of two polypeptide sequences or polynucleotide sequences, respectively.

Two or more polynucleotide sequences can be compared by determining their "percent identity." Two or more amino acid sequences likewise can be compared by determining their "percent identity." The percent identity of two sequences, whether nucleic acid or peptide sequences, is generally described as the number of exact matches between two aligned sequences divided by the length of the shorter sequence and multiplied by 100. An approximate alignment for nucleic acid sequences is provided by the local homology algorithm of Smith and Waterman, Advances in Applied Mathematics 2:482-489 (1981). This algorithm can be extended to use with peptide sequences using the scoring matrix developed by Dayhoff, Atlas of Protein Sequences and Structure, M.O. Dayhoff ed., 5 suppl. 3:353-358, National Biomedical Research Foundation, Washington, D.C., USA, and normalized by Gribskov, Nucl. Acids Res. 14(6):6745-6763 (1986). An implementation of this algorithm for nucleic acid and peptide sequences is provided by the Genetics Computer Group (Madison, WI) in their BestFit utility application. The default parameters for this method are described in the Wisconsin Sequence Analysis Package Program Manual, Version 8 (1995) (available from Genetics Computer Group, Madison, WI). Other equally suitable programs for calculating the percent identity or similarity between sequences are generally known in the art.

For example, percent identity of a particular nucleotide sequence to a reference sequence can be determined using the homology algorithm of Smith and Waterman with a default scoring table and a gap penalty of six nucleotide positions. Another method of establishing percent identity in the context of the present invention is to use the MPSRCH package of programs copyrighted by the University of Edinburgh, developed by John F. Collins and Shane S. Sturrok, and distributed by IntelliGenetics, Inc. (Mountain View, CA). From this suite of packages, the Smith-Waterman algorithm can be employed where default parameters are used for the scoring table (for example, gap open penalty of 12, gap extension penalty of one, and a gap of six). From the data generated, the "Match" value reflects "sequence identity." Other suitable programs for calculating the percent identity or similarity between sequences are generally known in the art, such as the alignment program BLAST, which can also be used with default parameters. For example, in a preferred embodiment, BLASTN and BLASTP can be used with the following default parameters for nucleic acid searches -- genetic code = standard; filter = none; strand = both; cutoff = 60; expect =10; Matrix = BLOSUM62; Descriptions = 50 sequences; sort by = HIGH SCORE; Databases = non-redundant, GenBank + EMBL + DDBJ + PDB + GenBank CDS translations + Swiss protein + Spupdate + PIR; (ii) polypeptide searches --. Details of these programs can be found at the following internet address: www.ncbi.nlm.gov/cgi-bin/BLAST.

Protein similarity and percent identity sequence searches can be carried out, for example, using Smith-Waterman Similarity Search algorithms (e.g., at www.ncbi.nlm.gov, or from commercial sources, such as, TimeLogic Corporation, Crystal Bay, NV). For example, in a preferred embodiment, the Smith-Waterman Similarity Search can be used with default parameters, for example, as follows: Weight MATRIX = BLOSUM62.MAA; Gap Opening PENALTY = -12; Gap Extension PENALTY = -2; FRAME PENALTY = 0; QUERY FORMAT = FASTA/PEARSON; QUERY TYPE = AA; QUERY SEARCH =1; QUERY SET = CGI_1d82ws301bde.seq; TARGET TYPE = AA; TARGET SET = NRPdb gsaa; SIGNIFICANCE = GAPPED; MAX SCORES = 30; MAX ALIGNMENTS = 20; Reporting THRESHOLD = Score=1; ALIGNMENT THRESHOLD = 20.

One of skill in the art can readily determine the proper search parameters to use for a given sequence, exemplary preferred Smith Waterman based parameters are presented above. For example, the search parameters may vary based on the size of the sequence in question. Thus, for polynucleotide sequences of the present invention the length of the polynucleotide sequence disclosed herein is searched against a selected database and compared to sequences of essentially the same length to determine percent identity. For example, a representative embodiment of the present invention would include an isolated polynucleotide comprising X contiguous nucleotides, wherein (i) the X contiguous nucleotides have at least about a selected level of percent identity relative to Y contiguous nucleotides of one or more of the sequences described herein or fragment thereof, and (ii) for search purposes X equals Y, wherein Y is a selected reference polynucleotide of defmed length (for example, a length of from 15 nucleotides up to the number of nucleotides present in a selected full-length sequence).

The sequences of the present invention can include fragments of the sequences, for example, from about 15 nucleotides up to the number of nucleotides present in the full-length sequences described herein, including all integer values falling within the above-described range. For example, fragments of the polynucleotide sequences of the present invention may be 30-60 nucleotides, 60-120 nucleotides, 120-240 nucleotides, 240-480 nucleotides, 480-1000 nucleotides, and all integer values therebetween.

The synthetic polynucleotides described herein include related polynucleotide sequences having about 80% to 100%, greater than 80-85%, preferably greater than 90-92%, more preferably greater than 95%, and most preferably greater than 98% up to 100% (including all integer values falling within these described ranges) sequence identity to the synthetic polynucleotide sequences disclosed herein when the sequences of the present invention are used as the query sequence against, for example, a database of sequences.

Two nucleic acid fragments are considered to "selectively hybridize" as described herein. The degree of sequence identity between two nucleic acid molecules affects the efficiency and strength of hybridization events between such molecules. A partially identical nucleic acid sequence will at least partially inhibit a completely identical sequence from hybridizing to a target molecule. Inhibition of hybridization of the completely identical sequence can be assessed using hybridization assays that are well known in the art (e.g., Southern blot, Northern blot, solution hybridization, or the like, see Sambrook, et al., *supra* or Ausubel et al., *supra*). Such assays can be conducted using varying degrees of selectivity, for example, using conditions varying from low to high stringency. If conditions of low stringency are employed, the absence of non-specific binding can be assessed using a secondary probe that lacks even a partial degree of sequence identity (for example, a probe having less than about 30% sequence identity with the target molecule), such that, in the absence of non-specific binding events, the secondary probe will not hybridize to the target.

When utilizing a hybridization-based detection system, a nucleic acid probe is chosen that is complementary to a target nucleic acid sequence, and then by selection of appropriate conditions the probe and the target sequence "selectively hybridize," or bind, to each other to form a hybrid molecule. A nucleic acid molecule that is capable of hybridizing selectively to a target sequence under "moderately stringent" typically hybridizes under conditions that allow detection of a target nucleic acid sequence of at least about 10-14 nucleotides in length having at least approximately 70% sequence identity with the sequence of the selected nucleic acid probe. Stringent hybridization conditions typically allow detection of target nucleic acid sequences of at least about 10-14 nucleotides in length having a sequence identity of greater than about 90-95% with the sequence of the selected nucleic acid probe. Hybridization conditions useful for probe/target hybridization where the probe and target have a specific degree of sequence identity, can be determined as is known in the art (see, for example, Nucleic Acid Hybridization: A Practical Approach, editors B.D. Hames and S.J. Higgins, (1985) Oxford; Washington, DC; IRL Press).

With respect to stringency conditions for hybridization, it is well known in the art that numerous equivalent conditions can be employed to establish a particular stringency by varying, for example, the following factors: the length and nature of probe and target sequences, base composition of the various sequences, concentrations of salts and other hybridization solution components, the presence or absence of blocking agents in the hybridization solutions (e.g., formamide, dextran sulfate, and polyethylene glycol), hybridization reaction temperature and time parameters, as well as, varying wash conditions. The selection of a particular set of hybridization conditions is selected following standard methods in the art (see, for example, Sambrook, et al., *supra* or Ausubel et al., *supra*).

A first polynucleotide is "derived from" second polynucleotide if the first polynucleotide has the same basepair sequence as a region of the second polynucleotide, its cDNA, complements thereof, or if the first polynucleotide displays substantial sequence identity to a region of the second polynucleotide, its cDNA, complements thereof, wherein sequence identity is determined as described above. Substantial sequence identity is typically about 90% or greater, preferably about 95% or greater, more preferably about 98% or greater.

A first polypeptide is "derived from" a second polypeptide if it is encoded by a first polynucleotide derived from a second polynucleotide, or the first polypeptide has the same amino acid sequence as the second polypeptide or a portion thereof, or the first polypeptide displays substantial sequence identity to the second polypeptide or a portion thereof, wherein sequence identity is determined as described above. Substantial sequence identity is typically about 90% or greater, preferably about 95% or greater, more preferably about 98% or greater.

Generally, a viral polypeptide is "derived from" a particular polypeptide of a virus (viral polypeptide) if it is (i) encoded by the same open reading frame of a polynucleotide of that virus (viral polynucleotide), or (ii) displays substantial sequence identity to a polypeptide of that virus as described above.

A polypeptide is "derived from" an HIV subtype if it is derived from a polypeptide present in a member of the subtype, derived from a polypeptide encoded by a polynucleotide present in a member of the subtype, encoded by a polynucleotide that is derived from a polynucleotide present in a member of the subtype, or derived from a polypeptide encoded by a polynucleotide that is derived from a polynucleotide present in a member of the subtype.

A polypeptide is "derived from" an HIV strain if it is derived from a polypeptide present in a member of the strain, derived from a polypeptide encoded by a polynucleotide present in a member of the strain, encoded by a polynucleotide that is derived from a polynucleotide present in a member of the strain, or derived from a polypeptide encoded by a polynucleotide that is derived from a polynucleotide present in a member of the strain.

"Analogous polypeptides" refers to polypeptides that are encoded by, or derived from polypeptides encoded by, the same gene of the same organism but from different polynucleotide sources. In the context of the present invention, different polynucleotide sources could be different subtypes, different serotypes or different strains. Thus, for example, a Gag polypeptide from a Subtype B HIV would be an analogous polypeptide to a Gag polypeptide from a Subtype C HIV, or an envelope polypeptide derived from a first HIV-1 subtype, serotype, or strain would be an analogous polypeptide to an envelope polypeptide derived from a second HIV-1 subtype, serotype, or strain. Examples of types of analogous polypeptides that could be derived from different HIV-1 subtypes or strains include, the envelope polypeptides gp41, gp120, gp140, and gp160, all of which are considered analogous polypeptides. Further, such analogous polypeptides may each comprise different alterations or mutations, for example, analogous polypeptides derived from the HIV-1 envelope gene include, but are not limited to, the following: a gp41 polypeptide, a gp120 polypeptide, a gp140 polypeptide, a gp160 polypeptide, a gp140 comprising a deletion of a portion of the V1 loop, a gp140 polypeptide comprising a deletion of a portion of the V2 loop, a gp 140 polypeptide comprising a deletion of a portion of the V3 loop, a gp140 polypeptide with a mutated protease cleavage site, a gp160 comprising a deletion of a portion of the V1 loop, a gp160 polypeptide comprising a deletion of a portion of the V2 loop, a gp 160 polypeptide comprising a deletion of a portion of the V3 loop, and a gp160 polypeptide with a mutated protease cleavage site.

A "gene" as used in the context of the present invention is a sequence of nucleotides in a genetic nucleic acid (viral genome, chromosome, plasmid, etc.) with which a genetic function is associated. A gene is a hereditary unit, for example of an organism comprising a polynucleotide sequence (e.g., an RNA sequence for HIV-1 or a proviral HIV-1 DNA sequence), that occupies a specific physical location (a "gene locus" or "genetic locus") within the genome of an organism. A gene can encode an expressed product, such as a polypeptide or a polynucleotide (e.g., tRNA). Alternatively, a gene may define a genomic location for a particular event/function, such as the binding of proteins and/or nucleic acids (e.g., 5' LTR), wherein the gene does not encode an expressed product. Examples of HIV-1 genes include, but are not limited to, Gag, Env, Pol (prot, RNase, Int), tat, rev, nef, vif, vpr, and vpu. A gene may include coding sequences, such as, polypeptide encoding sequences, and non-coding sequences, such as, promoter sequences, poly-adenlyation sequences, transcriptional regulatory sequences (e.g., enhancer sequences). Many eucaryotic genes have "exons" (coding sequences) interrupted by "introns" (non-coding sequences). In certain cases, a gene may share sequences with another gene(s) (e.g., overlapping genes). It is noted that in the general population, wild-type genes may include multiple prevalent versions that contain alterations in sequence relative to each other. These variations are designated "polymorphisms" or "allelic variations."

"Purified polynucleotide" refers to a polynucleotide of interest or fragment thereof that is essentially free, e.g., contains less than about 50%, preferably less than about 70%, and more preferably less than about 90%, of the protein with which the polynucleotide is naturally associated. Techniques for purifying polynucleotides of interest are well-known in the art and include, for example, disruption of the cell containing the polynucleotide with a chaotropic agent and separation of the polynucleotide(s) and proteins by ion-exchange chromatography, affinity chromatography and sedimentation according to density.

By "nucleic acid immunization" is meant the introduction of a nucleic acid molecule encoding one or more selected antigens into a host cell, for the *in vivo* expression of an antigen, antigens, an epitope, or epitopes. The nucleic acid molecule can be introduced directly into a recipient subject, such as by injection, inhalation, oral, intranasal and mucosal administration, or the like, or can be introduced *ex vivo,* into cells which have been removed from the host. In the latter case, the transformed cells are reintroduced into the subject where an immune response can be mounted against the antigen encoded by the nucleic acid molecule.

"Gene transfer" or "gene delivery" refers to methods or systems for reliably inserting nucleic acid (i.e., DNA or RNA) of interest into a host cell. Such methods can result in transient expression of non-integrated transferred DNA, extrachromosomal replication and expression of transferred replicons (e.g., episomes), or integration of transferred genetic material into the genomic DNA of host cells. Gene delivery expression vectors include, but are not limited to, vectors derived from adenoviruses, adeno-associated viruses, alphaviruses, herpes viruses, measles viruses, polio viruses, pox viruses, vesiculoviruses and vaccinia viruses. When used for immunization, such gene delivery expression vectors may be referred to as vaccines or vaccine vectors. In preferred embodiments, gene delivery vectors include both replicating and non replicating viral and bacterial vectors that serve as delivery vectors for polynucleotides encoding or expressing the polypeptides described herein.

The term "transfection" is used to refer to the uptake of foreign DNA by a cell. A cell has been "transfected" when exogenous DNA has been introduced inside the cell membrane. A number of transfection techniques are generally known in the art. *See, e*.*g*., Graham et al. (1973) Virology, 52:456*,* Sambrook et al. (1989) Molecular Cloning, a laboratory manual, Cold Spring Harbor Laboratories, New York, Davis et al. (1986) Basic Methods in Molecular Biology, Elsevier, and Chu et al. (1981) Gene 13:197. Such techniques can be used to introduce one or more exogenous DNA moieties into suitable host cells. The term refers to both stable and transient uptake of the genetic material, and includes uptake of peptide- or antibody-linked DNAs.

A "vector" is capable of transferring gene sequences to target cells (e.g., viral vectors, non-viral vectors, particulate carriers, and liposomes). Thus, the term includes bacterial, fungal as well as viral vectors.

"Lentiviral vector", and "recombinant lentiviral" refer to a nucleic acid construct which carries, and within certain embodiments, is capable of directing the expression of a nucleic acid molecule of interest. The lentiviral vector include at least one transcriptional promoter/enhancer or locus defining element(s), or other elements which control gene expression by other means such as alternate splicing, nuclear RNA export, post-translational modification of messenger, or post-transcriptional modification of protein. Such vector constructs must also include a packaging signal, long terminal repeats (LTRS) or portion thereof, and positive and negative strand primer binding sites appropriate to the retrovirus used (if these are not already present in the retroviral vector). Optionally, the recombinant lentiviral vector may also include a signal which directs polyadenylation, selectable markers such as Neo, TK, hygromycin, phleomycin, histidinol, or DHFR, as well as one or more restriction sites and a translation termination sequence. By way of example, such vectors typically include a 5' LTR, a tRNA binding site, a packaging signal, an origin of second strand DNA synthesis, and a 3'LTR or a portion thereof

"Lentiviral vector particle" as utilized within the present invention refers to a lentivirus which carries at least one gene of interest. The retrovirus may also contain a selectable marker. The recombinant lentivirus is capable of reverse transcribing its genetic material (RNA) into DNA and incorporating this genetic material into a host cell's DNA upon infection. Lentiviral vector particles may have a lentiviral envelope, a non-lentiviral envelope (e.g., an ampho or VSV-G envelope), or a chimeric envelope.

"Alphaviral vector", and "recombinant alphaviral vector" and "alphaviral replicon vector" refer to a nucleic acid construct which carries, and within certain embodiments, is capable of directing the expression of a nucleic acid molecule of interest. The alphaviral vector includes at least one transcriptional promoter/enhancer or other elements which control gene expression by other means such as alternate splicing, nuclear RNA export, post-translational modification of messenger, or post-transcriptional modification of protein. Such vector constructs must also include a packaging signal, and alphaviral replication recognition sequences. Optionally, the recombinant alphaviral vector may also include a signal which directs polyadenylation, selectable markers such as Neo, TK, hygromycin, phleomycin, histidinol, or DHFR, as well as one or more restriction sites and a translation termination sequence. Typically, the alphaviral vector will include coding sequences for the alphaviral non-structural proteins, a packaging site, replication recognition sequences and a sequence capable of directing the expression of the nucleic acid molecule of interest.

"Expression cassette" refers to an assembly which is capable of directing the expression of a sequence or gene of interest. An expression cassette typically includes a promoter which is operably linked to the polynucleotide sequences or gene(s) of interest. Other control elements may be present as well. Expression cassettes described herein may be contained within a plasmid construct. In addition to the components of the expression cassette, the plasmid construct may also include a bacterial origin of replication, one or more selectable markers, a signal which allows the plasmid construct to exist as single-stranded DNA (e.g., a M13 origin of replication), a multiple cloning site, and a "mammalian" origin of replication (e.g., a SV40 or adenovirus origin of replication).

"Replicon particle" or "recombinant particle" refers to a virion-like unit containing an alphavirus RNA vector replicon. Generally, recombinant particles comprises one or more viral structural proteins, a lipid envelope and an RNA vector replicon. Preferably, the recombinant particle contains a nucleocapsid structure that is contained within a host cell-derived lipid bilayer, such as a plasma membrane, in which one or more viral envelope glycoproteins (e.g., E2, E1) are embedded. The particle may also contain other components (*e*.*g*., targeting elements such as biotin, other viral structural proteins or portions thereof, hybrid envelopes, or other receptor binding ligands).

"Packaging cell" refers to a cell that comprises those elements necessary for production of infectious recombinant viral vector, but which lack the recombinant viral vector. Typically, such packaging cells contain one or more expression cassettes that are capable of expressing proteins necessary for the replication and packaging of an introduced vector, for example, in the case of a lentiviral vector expression cassettes which encode *Gag, pol and* env proteins, in the case of an alphaviral vector, expression cassettes that encode alphaviral structural proteins.

"Producer cell" or "vector producing cell" refers to a cell which contains all elements necessary for production of recombinant viral vector particles.

Transfer of a "suicide gene" (e.g., a drug-susceptibility gene) to a target cell renders the cell sensitive to compounds or compositions that are relatively nontoxic to normal cells. Moolten, F.L. (1994) Cancer Gene Ther. 1:279-287. Examples of suicide genes are thymidine kinase of herpes simplex virus (HSV-tk), cytochrome P450 (Manome et al. (1996) Gene Therapy 3:513-520), human deoxycytidine kinase (Manome et al. (1996) Nature Medicine 2(5):567-573) and the bacterial enzyme cytosine deaminase (Dong et al. (1996) Human Gene Therapy 7:713-720). Cells which express these genes are rendered sensitive to the effects of the relatively nontoxic prodrugs ganciclovir (HSV-tk), cyclophosphamide (cytochrome P450 2B1), cytosine arabinoside (human deoxycytidine kinase) or 5-fluorocytosine (bacterial cytosine deaminase). Culver et al. (1992) Science 256:1550-1552, Huber et al. (1994) Proc. Natl. Acad. Sci. USA 91:8302-8306.

A "selectable marker" or "reporter marker" refers to a nucleotide sequence included in a gene transfer vector that has no therapeutic activity, but rather is included to allow for simpler preparation, manufacturing, characterization or testing of the gene transfer vector.

A "specific binding agent" refers to a member of a specific binding pair of molecules wherein one of the molecules specifically binds to the second molecule through chemical and/or physical means. One example of a specific binding agent is an antibody directed against a selected antigen.

By "subject" is meant any member of the subphylum chordata, including, without limitation, humans and other primates, including non-human primates such as baboons, rhesus macaque, chimpanzees and other apes and monkey species; farm animals such as cattle, sheep, pigs, goats and horses; domestic mammals such as dogs and cats; laboratory animals including rodents such as mice, rats, rabbits, and guinea pigs; birds, including domestic, wild and game birds such as chickens, turkeys and other gallinaceous birds, ducks, geese, and the like. The term does not denote a particular age. Thus, both adult and newborn individuals are intended to be covered. The system described above is intended for use in any of the above vertebrate species, since the immune systems of all of these vertebrates operate similarly.

By "subtype" is meant a phylogenetic classification of similar organisms into groups based on similarities at the genetic (i.e., nucleic acid sequence) level. Such groups are designated "subtypes." In the HIV field, a well known and widely accepted centralized organization for the determination of such similarities and classification of particular viral isolates into subtypes is the Los Alamos National Laboratory. The HIV subtypes referred to herein are those as determined by the Los Alamos National Laboratory. (See, e.g., Myers, et al., Los Alamos Database, Los Alamos National Laboratory, Los Alamos, New Mexico; Myers, et al., Human Retroviruses and Aids, 1990, Los Alamos, New Mexico: Los Alamos National Laboratory.) A subtype can also be referred to as a "clade." The term "subtypes" includes the subtypes currently identified as well as circulating recombinant forms (CRFs). HIV subtypes (including CRFs) are continually being characterized and can be found on the HIV database from Los Alamos National Laboratories, available on the internet. Thus, subtypes include subtypes A (e.g., A1, A2), B, C, D, F (e.g., F1, F2), G, H, J and K, as well as various CRFs.

By "serotype" is meant a classification of similar organisms based on antibody cross-reactivity.

By "strain" is intended an organism from within the subtype but which is differentiated from other members of the same subtype based on differences in nucleic acid sequence.

By "pharmaceutically acceptable" or "pharmacologically acceptable" is meant a material which is not biologically or otherwise undesirable, i.e., the material may be administered to an individual in a formulation or composition without causing any undesirable biological effects or interacting in a deleterious manner with any of the components of the composition in which it is contained.

By "physiological pH" or a "pH in the physiological range" is meant a pH in the range of approximately 7.0 to 8.0 inclusive, more typically in the range of approximately 7.2 to 7.6 inclusive.

As used herein, "treatment" refers to any of (i) the prevention of infection or reinfection, as in a traditional vaccine, (ii) the reduction or elimination of symptoms, or (iii) the substantial or complete elimination of the pathogen in question. Treatment may be effected prophylactically (prior to infection) or therapeutically (following infection).

By "co-administration" is meant administration of more than one composition, component of a composition, or molecule. Thus, co-administration includes concurrent administration or sequentially administration (in any order), via the same or different routes of administration. Non-limiting examples of co-administration regimes include, co-administration of nucleic acid and polypeptide; co-administration of different nucleic acids (e.g., different expression cassettes as described herein and/or different gene delivery vectors); and co-administration of different polypeptides (e.g., different HIV polypeptides and/or different adjuvants). The term also encompasses multiple administrations of one of the co-administered molecules or compositions (*e*.*g*., multiple administrations of one or more of the expression cassettes described herein followed by one or more administrations of a polypeptide-containing composition). In cases where the molecules or compositions are delivered sequentially, the time between each administration can be readily determined by one of skill in the art in view of the teachings herein.

The compositions may be given more than once (e.g., a "prime" administration followed by one or more "boosts") to achieve the desired effects. The same composition can be administered as the prime and as the one or more boosts. Alternatively, different compositions can be used for priming and boosting. For example, in certain embodiments, multiple immunizations (primes and/or boosts) of polypeptide compositions are administered.

"T lymphocytes" or "T cells" are non-antibody producing lymphocytes that constitute a part of the cell-mediated arm of the immune system. T cells arise from immature lymphocytes that migrate from the bone marrow to the thymus, where they undergo a maturation process under the direction of thymic hormones. Here, the mature lymphocytes rapidly divide increasing to very large numbers. The maturing T cells become immunocompetent based on their ability to recognize and bind a specific antigen. Activation of immunocompetent T cells is triggered when an antigen binds to the lymphocyte's surface receptors.

### 2.0.0 MODES OF CARRYING OUT THE INVENTION

Before describing the present invention in detail, it is to be understood that this invention is not limited to particular formulations or process parameters as such may, of course, vary. It is also to be understood that the terminology used herein is for the purpose of describing particular embodiments of the invention only, and is not intended to be limiting.

Although a number of methods and materials similar or equivalent to those described herein can be used in the practice of the present invention, the preferred materials and methods are described herein.

### 2.1.0 GENERAL OVERVIEW OF THE INVENTION

The present invention relates to combination approaches to generate immune responses in subjects using compositions comprising immunogenic polynucleotides and polypeptides.

In one general aspect of the present invention, two or more gene delivery vectors, each vector comprising, or consisting essentially of, one polynucleotide encoding an identical or analogous immunogenic polypeptide derived from a microorganism (e.g., virus, bacteria, fungi, etc.) are used to generate an immune response in a subject. The gene delivery vectors may be viral or non-viral. In some embodiments, the gene delivery vectors are adenovirus or alphavirus vectors.

One or more of the gene delivery vectors may comprise further additional components, such as immune enhancers, immunoregulatory components, carriers, particles, excipients, expression control sequences, etc. In addition, one or more of the gene delivery vectors may include further components such as molecules to enhance the immune response (e.g., liposomes, PLG, particles, alum, etc.).

Optionally, the methods also comprise administering a polypeptide component that comprises one or more immunogenic polypeptides identical or analogous to the polypeptide encoded by one or more of the gene delivery vectors. Further, one or more of the polypeptide components may comprise further components, such as, immune enhancers, immunoregulatory components, adjuvants, carriers, particles, excipients, etc.

In a second general aspect of the present invention, one or more of the gene delivery components comprises two or more polynucleotide sequences comprising coding sequences for two or more identical or analogous immunogenic polypeptides derived from a microorganism (e.g., virus, bacteria, fungi, etc.), wherein the coding sequences for at least two of the immunogenic polypeptides are derived from different subtypes, serotypes, or strains of the microorganism.

In any of these aspects, the optional polypeptide component may comprise one or more immunogenic polypeptides identical or analogous to the polypeptide encoded by the gene delivery vector that encodes two more identical or analogous immunogenic polypeptides. The polypeptide component may provide less than, greater than or the same number of identical or analogous immunogenic polypeptides encoded by one or both gene delivery vectors. Furthermore, the immunogenic polypeptides of the polypeptide composition may be derived from the same and/or different subtypes, serotypes, or strains, as the immunogenic polypeptides provided by the gene delivery vectors.

The gene delivery vector(s) as described herein may comprise further components, such as immune enhancers, immunoregulatory components, carriers, particles, excipients, expression control sequences, etc. In addition, the gene delivery vectors may include further components such as molecules to enhance the immune response (e.g., liposomes, PLG, particles, alum, etc.). Further, the polypeptide component may comprise further components, such as, immune enhancers, immunoregulatory components, adjuvants, carriers, particles, excipients, etc.

The invention is exemplified herein with reference to Human Immunodeficiency Virus 1 (HIV-1). One of ordinary skill in the art, in view of the teachings of the present specification, can apply the teachings of the present invention to other suitable organisms, for example, microorganisms. The compositions and methods of the present invention may, for example, employ polynucleotides encoding HIV envelope polypeptides and well as HIV envelope polypeptides, e.g., HIV envelope proteins identical or analogous to those encoded by the polynucleotides, to induce broad and/or potent neutralizing activity against diverse HIV strains. Although described with reference to the HIV virus, the compositions and methods of the present invention can be applied to other virus families having a variety of subtypes, serotypes, and/or strain variations, for example, including but not limited to other non-HIV retroviruses (e.g. HTLV-1, 2), hepadnoviruses (e.g. HBV), herpesviruses (e.g. HSV-1,2, CMV, EBV, varizellazoster, etc.), flaviviruses (e.g. HCV, Yellow fever, Tick borne encephalitis, St. Louis Encephalitis, West Nile Virus, etc.), coronaviruses (e.g. SARS), paramyxoviruses (e.g., PIV, RSV, measles etc.), influenza viruses, picornaviruses, reoviruses (e.g., rotavirus), arenaviruses, rhabdoviruses, papovaviruses, parvoviruses, adenoviruses, Dengue virus, bunyaviruses (e.g., hantavirus), calciviruses (e.g. Norwalk virus), filoviruses (e.g. , Ebola, Marburg).

The diversity and mutability of the HIV virus present challenges to HIV vaccine development. HIV continues to spread globally, with upwards of 42 million people infected with HIV (UNAIDS Report on the global HIV/AIDS epidemic, UNAIDS, Geneva, Switzerland (December 2002). These people are infected with different HIV subtypes (and/or strains). The infecting HIV subtype (and/or strain) is typically geographically dependent. In one aspect, the present invention relates to compositions and methods that provide the ability to induce broad and potent neutralizing antibodies against the diverse HIV subtypes, serotypes, and/or strains for the treatment of infections, reduction of infection risk, reduction of transmission, reduction of disease manifestations, and/or prevention of HIV infections arising in different regions.

The approaches described herein may induce potent and broad HIV-neutralization activity. The approaches include immunization with a variety of polynucleotides encoding HIV polypeptides derived from different subtypes, serotypes, or strains combined with immunization using HIV polypeptides derived from different subtypes, serotypes, or strains. The invention further includes immunization using various doses and immunization regimens of such polynucleotides and polypeptides.

Accordingly, in a first aspect of the present invention, one or more of the gene delivery vectors (*e*.*g*., alphavirus or adenovirus gene delivery vectors) of the present invention each comprise, or consist essentially of, one polynucleotide encoding an identical or analogous HIV immunogenic polypeptide and necessary vector sequences. The optional polypeptide component comprises of one or more HIV immunogenic polypeptides identical or analogous to one or more of the polypeptides encoded by said polynucleotide component. In one embodiment, at least one HIV immunogenic polypeptide of the polypeptide component is derived from a different HIV subtype, serotype, or strain than the coding sequence of at least one of the immunogenic polypeptides encoded by the polynucleotide components. In this context, consists essentially of refers to the presence of one polynucleotide sequence encoding one HIV immunogenic polypeptide in the polynucleotide compositions.

In preferred embodiments, the HIV immunogenic polypeptides encoded by the polynucleotides of the two or more gene delivery vectors are identical or analogous. For example, in one embodiment of the present invention, the HIV immunogenic polypeptide encoded by at least one of the polynucleotide components is derived from subtype B, and the HIV immunogenic polypeptide encoded by at least one of the other polynucleotide components is derived from subtype C. Likewise, when present, the optional polypeptide component may be derived from any subtype, strain or isolate (e.g., subtype B, subtype C or other subtypes).

Also described herein are methods for generating an immune response in a mammal, the methods comprising: administering to the mammal first and second gene delivery vectors, each gene delivery vector comprising a polynucleotide encoding an HIV immunogenic polypeptide. In certain embodiments, the first and second gene delivery vehicles are different, for example alphavirus vectors and adenovirus (replicating or nonreplicating) vectors. The gene delivery vectors can be administered concurrently or sequentially. The first and second gene delivery vectors may encode HIV immunogenic polypeptides from the same HIV subtype, strain or serotype or, alternatively, may encode HIV polypeptides derived from different HIV subtypes, serotypes, or strains. In addition, the first and second gene delivery vectors may encode identical or analogous HIV polypeptides. In one embodiment of the present invention, the analogous HIV immunogenic polypeptides coding sequences of the first gene delivery vector may be derived from different subtypes of HIV than the sequences of the second gene delivery vector. In another embodiment, the analogous HIV polypeptides encoded by polynucleotides of the first and second gene delivery vectors may derived from different strains of HIV from the same HIV subtype.

The gene delivery vectors described herein may be administered concurrently or sequentially. For example, sequential administration may be priming and boosting administration, i.e., a first gene delivery vector comprising polynucleotide encoding an immunogenic HIV polypeptide is used for immunization via delivery of the polynucleotide (e.g., a prime) and a second gene delivery vector different from the first gene delivery vector is used for immunization with an identical or analogous immunogenic HIV polypeptide derived from the same or a different HIV subtype, serotype, or strain (e.g., a boost).

Various prime-boost regimens have been described in the art and are well known to those of ordinary skill. In a typical prime-boost regimen, a first component providing a polypeptide immunogen (*e*.*g*., first gene delivery vector encoding an HIV immunogenic polypeptide) is administered to a subject; the initial immune response is measured (e.g., by determining the production of binding antibodies to the encoded immunogen for a humoral immune response) in said subject until the titer of binding antibodies begins to decline; and a second component (e.g., second gene delivery vector encoding an identical or analogous HIV immunogenic polypeptide) providing a second but related polypeptide immunogen is administered to the subject. In preferred embodiments, the priming gene delivery vector is a replicating adenovirus vector, a nonreplicating adenovirus vector or a nonreplicating alphavirus vector and the boosting gene delivery vector is a nonreplicating adenovirus or nonreplicating alphavirus vector.

For example, a first gene delivery vector may be used for a priming nucleic acid immunization, wherein the first polynucleotide molecule of the first gene delivery vector encodes an HIV gp140 envelope polypeptide (i) derived from a South African HIV subtype C isolate/strain, (ii) that is codon optimized for expression in mammalian cells, and (iii) is mutated by deletion of the V2 loop (e.g., gp140mod.TV1.de1V2, as described for example in PCT International Publication No. WO/02/04493). Administration of the first gene delivery vector is followed by administration of at least a second (boosting) gene delivery vector, the second gene delivery vector comprising a polynucleotide encoding an HIV gp140 envelope polypeptide, which may or may not include mutations contained in the first polynucleotide (e.g., a polynucleotide encoding
gp140.mut7.modSF162.de1V2, as described for example in PCT International Publication No. WO/00/39302); and a different (non-gp140 Env polypeptide), for example an HIV Gag, Pol, RT, Tat, Rev and/or Nef polypeptide from the same or different strain. Oligomeric forms of the envelope polypeptide may be used (e.g., o-gp140 as described in PCT International Publication No. WO/00/39302 and US Patent No. 6,602,705).

Further, a single prime may be followed by multiple boosts, multiple primes may be followed by a single boost, multiple primes may be followed by multiple boosts, or a series of primes and boosts may be used.

In yet another embodiment, the methods described herein are used to broadly raise neutralizing antibodies against viral strains that use the CCR5 coreceptor for cell entry. For example, a composition for generating neutralizing antibodies in a mammal may comprise, a first gene delivery vector comprising, or consisting essentially of, one polynucleotide encoding an HIV immunogenic polypeptide derived from an HIV strain that uses the CCR5 coreceptor for cell entry, and a second gene delivery vector encoding one or more HIV immunogenic polypeptides derived from an HIV strain that uses the CCR5 coreceptor for cell entry analogous to the polypeptide encoded by said first gene delivery vector. In certain embodiments, the HIV immunogenic polypeptide encoded by the second gene delivery vector is derived from a different HIV strain than the first gene delivery vector. In other embodiments, the second gene delivery vector encodes more than one HIV immunogenic polypeptide, which polypeptide coding sequences are derived from more than one HIV strain that uses the CCR5 coreceptor for cell entry.

Additional gene delivery vectors may also be administered, for example, one or more gene delivery vectors comprising polynucleotides encoding analogous HIV polypeptides from different subtypes. For example, three gene delivery vectors may be administered concurrently or sequentially, wherein the gene delivery vectors encode three immunogenic HIV polypeptides, one coding sequence derived from a subtype B strain, one coding sequence derived from a subtype C strain, and one coding sequence derived from a subtype E strain. The optional polypeptide component may comprise three immunogenic HIV polypeptides, one coding sequence derived from a subtype B strain, one coding sequence derived from a subtype C strain, and one coding sequence derived from a subtype O strain.

In another embodiment of this aspect of the present invention, the polynucleotides of the gene delivery vectors comprise polynucleotides encoding analogous HIV immunogenic polypeptides from different subtypes, serotypes, or strains as the polypeptides of the polypeptide component. For example, DNA immunization with two or more DNA molecules encoding HIV gp140 polypeptides (wherein the two or more gp140 coding sequences are derived from two or more HIV-1 subtypes, serotypes, or strains). The optional polypeptide component, used for protein immunization, comprises two or more gp140 polypeptides (wherein the two or more gp140 coding sequences are derived from two or more HIV-1 subtypes, serotypes, or strains, with the proviso that at least one of the polypeptide sequences is derived from an HIV-1 subtype, serotype, or strain not represented in the DNA component).

In a further aspect, the present invention relates to the use of varied doses of polynucleotides and optional polypeptides in prime/boost methods, particularly the methods described herein. In any immunization method using, for example, a mixed polynucleotide prime (i.e., two or more polynucleotides encoding immunogenic HIV polypeptides derived from two or more HIV subtypes, serotypes, or strains) in conjunction with a polypeptide boost the present invention includes using reduced doses of each single component to provide an equivalent immune response to using full doses of each component. In one embodiment, the high threshold of DNA is the maximum tolerable dose of DNA (e.g., about 5 mg to about 10 mg total DNA), the low threshold of DNA is the minimum effective dose (e.g., about 2 ug to about 10 ug total DNA), the high threshold of protein is the maximum tolerable dose of protein (e.g., about 1 mg total protein), the low threshold of protein is the minimum effective dose (e.g., about 2 ug total protein). Furthermore, the total DNA dose may be divided among the polynucleotides of the polynucleotide component. Further, the total polypeptide dose may be divided among the polypeptides comprising the polypeptide component. The total DNA and total protein are both typically above the low threshold values.

In a preferred embodiment, the total amount of DNA in a given DNA immunization has a high threshold of less than or equal to about 10 mg total DNA and greater than or equal to 1 mg total DNA, and the total amount of protein in a given polypeptide boost has a high threshold of less than or equal to about 200 ug total protein product and greater than or equal to 10 ug of total protein. For example, when administering two gene delivery vectors each encoding an immunogenic HIV polypeptide the dose of each DNA molecule per subject may be one milligram of each DNA molecule encoding an immunogenic HIV polypeptide, for a total of 2 mg for the two DNA molecules, or 0.5 mg of each DNA molecule encoding an immunogenic HIV polypeptide, for a total of 1 mg for the two DNA molecules.

Dosing with the optional polypeptide component may be similarly varied, for example, using a polypeptide component having two immunogenic HIV polypeptides the dose of each polypeptide per subject may be 100 micrograms of each immunogenic HIV polypeptide, for a total of 200 ug for the two polypeptides, 50 micrograms of each immunogenic HIV polypeptide, for a total of 100 ug for the two polypeptides, or 25 ug of each immunogenic HIV polypeptide, for a total of 50 ug for the two polypeptides. As described above, more than two polypeptides may be included in the polypeptide component of the present invention.

Exemplary polynucleotides included in the gene delivery vectors, methods of making these polynucleotides and constructs, corresponding polypeptide products, and methods of making polypeptides useful for HIV immunization have been previously described, for example, in the following PCT International Publication Nos.: WO/00/39302; WO/00/39303; WO/00/39304; WO/02/04493; WO/03/004657; WO/03/004620; and WO/03/020876.

Although described generally with reference to HIV subtypes B and C as exemplary subtypes, the compositions and methods of the present invention are applicable to a wide variety of HIV subtypes, serotypes, or strains and immunogenic polypeptides encoded thereby, including but not limited to the previously identified HIV-1 subtypes A through K, N and O, the identified CRFs (circulating recombinant forms), and HIV-2 strains and its subtypes. See, e.g., Myers, et al., Los Alamos Database, Los Alamos National Laboratory, Los Alamos, New Mexico; Myers, et al., Human Retroviruses and Aids, 1990, Los Alamos, New Mexico: Los Alamos National Laboratory. Further, the compositions and methods of the present invention may be used to raise broadly reactive neutralizing antibodies against viral strains and subtypes that use the CCR5 coreceptor for cell entry (for example, both TV1 and SF162 use the CCR5 coreceptor).

The optional polypeptide component of the present invention may comprise fragments of immunogenic polypeptide, for example, wherein the polypeptide sequence or a portion thereof contains an amino acid sequence of at least 3 to 5 amino acids, more preferably at least 8 to 10 amino acids, and even more preferably at least 15 to 20 amino acids from a polypeptide encoded by the nucleic acid sequence. Also encompassed are polypeptide sequences that are immunologically identifiable with a polypeptide encoded by the sequence. Further, polyproteins can be constructed by fusing in-frame two or more polynucleotide sequences encoding polypeptide or peptide products.

In addition, the polynucleotides of the gene delivery components of the present invention may comprise one or more monocistronic expression cassettes comprising polynucleotides encoding immunogenic HIV polypeptides, or one or more polycistronic expression cassettes comprising polynucleotides encoding immunogenic HIV polypeptides, or combinations thereof. Polycistronic coding sequences may be produced, for example, by placing two or more polynucleotide sequences encoding polypeptide products adjacent each other, typically under the control of one promoter, wherein each polypeptide coding sequence may be modified to include sequences for internal ribosome binding sites.

A variety of combinations of polynucleotides encoding immunogenic polypeptides (e.g., HIV immunogenic polypeptides) and immunogenic polypeptides or fragments thereof (e.g., HIV immunogenic polypeptides) can be used in the practice of the present invention. Polynucleotide sequences encoding immunogenic polypeptides can be included in a polynucleotide component of compositions of the present invention, for example, as DNA immunization constructs containing, for example, a synthetic Env expression cassettes, a synthetic Gag expression cassette, a synthetic pol-derived polypeptide expression cassette, a synthetic expression cassette comprising sequences encoding one or more accessory or regulatory genes (e.g., tat, rev, nef, vif, vpu, vpr). Immunogenic polypeptides may be included as purified polypeptides in the polypeptide component of compositions of the present invention.

The immunogenic polypeptides may be synthetic or wild-type. In preferred embodiments the immunogenic polypeptides are antigenic viral proteins, or fragments thereof.

### 2.2.0 IDENTIFICATION OF ANALOGOUS POLYPEPTIDES AND POLYNUCLEOTIDES ENCODING SUCH POLYPEPTIDES

The compositions and methods of the present invention are described with reference to exemplary HIV-1 sequences. The present invention is not limited to the sequences described herein. Numerous sequences for use in the practice of the present invention have been previously described (see, e.g., PCT International Publication Nos. WO/00/39302; WO/00/39303; WO/00/39304; WO/02/04493; WO/03/004657; WO/03/004620; and WO/03/020876.). Typically, the polynucleotide sequences used in the practice of the present invention encode polypeptides derived from a viral source (e.g., HIV-1). The polypeptides are typically derived from antigenic viral proteins, in particular, group specific antigen polypeptides, envelope polypeptides, capsid polypeptides, and other structural and non-structural polypeptides. The present invention is particularly described with reference to the use of envelope polypeptides and modifications thereof (and polynucleotides encoding same) derived from various subtypes, serotypes, or strains of the HIV-1 virus. Other HIV-1 polypeptides and polynucleotides encoding such polypeptides may be used in the practice of the present invention including, but not limited to, Gag, Pol (including Protease, Reverse Transcriptase, and Integrase), Tat, Rev, Nef, Vif, Vpr, and Vpu.

The HIV genome and various polypeptide-encodhig regions are shown in Table 1. The nucleotide positions are given relative to an HIV-1 Subtype C isolate from South Africa strain 8_5_TV1_C.ZA. However, it will be readily apparent to one of ordinary skill in the art in view of the teachings of the present disclosure how to determine corresponding regions in other HIV strains (from the same or different subtypes) or variants (*e.g.*, isolates HIV_{IIIb}, HIV_{SF2}, HIV-1_{SF162}, HIV-1_{SF170}, HIV_{LAV}, HIV_{LAI} HIV_{MN}, HIV-1_{CM235}, HIV-1_{US4}, other HIV-1 strains from diverse subtypes(e.g., subtypes, A through K, N and O), the identified CRFs (circulating recombinant forms), HIV-2 strains and diverse subtypes and strains (e.g., HIV-2_{UC1} and HIV-2_{UC2}), and simian immunodeficiency virus (SIV). (See, e.g., Virology, 3rd Edition (W.K. Joklik ed. 1988); Fundamental Virology, 2nd Edition (B.N. Fields and D.M. Knipe, eds. 1991); Virology, 3rd Edition (Fields, BN, DM Knipe, PM Howley, Editors, 1996, Lippincott-Raven, Philadelphia, PA; for a description of these and other related viruses), using for example, sequence comparison programs (e.g., BLAST and others described herein) or identification and alignment of structural features (*e*.*g*., a program such as the "ALB" program described herein that can identify the various regions).

**Table 1**

| **Regions of the HIV Genome relative to the Sequence of 8_5_TV1_C.ZA** | |
|---|---|
| **Region** | **Position in nucleotide sequence** |
| **5'LTR** | **1-636** |
| U3 | 1-457 |
| R | 458-553 |
| U5 | 554-636 |
| NFkB II | 340-348 |
| NFkB I | 354-362 |
| Sp1 III | 379-388 |
| Sp1 II | 390-398 |
| Sp1 I | 400-410 |
| TATA Box | 429-433 |
| TAR | 474-499 |
| Poly A signal | 529-534 |
| | |
| **PBS** | **638-655** |
| | |
| **p7 binding region, packaging signal** | **685-791** |
| **Gag:** | **792-2285** |
| p17 | 792-1178 |
| p24 | 1179-1871 |
| Cyclophilin A bdg. | 1395-1505 |
| MHR | 1632-1694 |
| p2 | 1872-1907 |
| p7 | 1908-2072 |
| Frameshift slip | 2072-2078 |
| p1 | 2073-2120 |
| p6Gag | 2121-2285 |
| Zn-motif I | 1950-1991 |
| Zn-motif II | 2013-2054 |
| **Pol:** | **2072-5086** |
| p6Pol | 2072-2245 |
| Prot | 2246-2542 |
| p66RT | 2543-4210 |
| p15RNaseH | 3857-4210 |
| p31Int | 4211-5086 |
| **Vif:** | **5034-5612** |
| Hydrophilic region | 5292-5315 |
| **Vpr:** | **5552-5839** |
| Oligomerization | 5552-5677 |
| Amphipathic a-helix | 5597-5653 |
| **Tat:** | **5823-6038 and 8417-8509** |
| Tat-1 exon | 5823-6038 |
| Tat-2 exon | 8417-8509 |
| N-terminal domain | 5823-5885 |
| Trans-activation domain | 5886-5933 |
| Transduction domain | 5961-5993 |
| **Rev:** | **5962-6037 and 8416-8663** |
| Rev-1 exon | 5962-6037 |
| Rev-2 exon | 8416-8663 |
| High-affinity bdg. site | 8439-8486 |
| Leu-rich effector domain | 8562-8588 |
| **Vpu:** | **6060-6326** |
| Transmembrane domain | 6060-6161 |
| Cytoplasmic domain | 6162-6326 |
| **Env (gp160):** | **6244-8853** |
| Signal peptide | 6244-6324 |
| gp120 | 6325-7794 |
| V1 | 6628-6729 |
| V2 | 6727-6852 |
| V3 | 7150-7254 |
| V4 | 7411-7506 |
| V5 | 7663-7674 |
| C1 | 6325-6627 |
| C2 | 6853-7149 |
| C3 | 7255-7410 |
| C4 | 7507-7662 |
| C5 | 7675-7794 |
| CD4 binding | 7540-7566 |
| gp41 | 7795-8853 |
| Fusion peptide | 7789-7842 |
| Oligomerization domain | 7924-7959 |
| N-terminal heptad repeat | 7921-8028 |
| C-terminal heptad repeat | 8173-8280 |
| Immunodominant region | 8023-8076 |
| **Nef:** | **8855-9478** |
| Myristoylation | 8858-8875 |
| SH3 binding | 9062-9091 |
| Polypurine tract | 9128-9154 |
| SH3 binding | 9296-9307 |

It will be readily apparent that one of skill in the art can align any HIV sequence to that shown in Table 1 to determine relative locations of any particular HIV gene. For example, using one of the alignment programs described herein (*e*.*g*., BLAST), other HIV genomic sequences can be aligned with 8_5_TV1__C.ZA (Table 1) and locations of genes determined. Polypeptide sequences can be similarly aligned. As described in detail in International Publication No. WO/00/39303, Env polypeptides (e.g., gp120, gp140 and gp160) include a "bridging sheet" comprised of 4 anti-parallel beta-strands (beta-2, beta-3, beta -20 and beta -21) that form a beta -sheet. Extruding from one pair of the beta -strands (beta -2 and beta -3) are two loops, V1 and V2. The beta -2 sheet occurs at approximately amino acid residue 113 (Cys) to amino acid residue 117 (Thr) while beta -3 occurs at approximately amino acid residue 192 (Ser) to amino acid residue 194 (Ile), relative to SF-162. The "V1/V2 region" occurs at approximately amino acid positions 120 (Cys) to residue 189 (Cys), relative to SF-162. Extruding from the second pair of beta -strands (beta -20 and beta -21) is a "small-loop" structure, also referred to herein as "the bridging sheet small loop." The locations of both the small loop and bridging sheet small loop can be determined relative to HXB-2 following the teachings herein and in PCT International Publication No. WO/00/39303.

### 2.3.0 EXPRESSION CASSETTES COMPRISING POLYNUCLEOTIDE SEQUENCES, VECTORS, POLYPEPTIDES, FURTHER COMPONENTS, AND FORMULATIONS USEFUL IN THE PRACTICE OF THE PRESENT INVENTION

Compositions for the generation of immune responses of the present invention comprise at least first and second gene delivery vectors, each gene delivery vector comprising a polynucleotide encoding an immunogenic viral polypeptides. Such polynucleotides may comprise native viral sequences encoding immunogenic viral polypeptides or synthetic polynucleotides encoding immunogenic polypeptides. Synthetic polynucleotides may include sequence optimization to provide improved expression of the encoded polypeptides relative to the analogous native polynucleotide sequences. Further, synthetic polynucleotides may comprise mutations (single or multiple point mutations, missense mutations, nonsense mutations, deletions, insertions, etc.) relative to corresponding wild-type sequences.

The optional polypeptide component of the compositions of the present invention may comprise one or more immunogenic viral polypeptide. Such polypeptides may comprise native immunogenic viral polypeptides or modified immunogenic polypeptides. Modified polypeptides may include sequence optimization to provide improved expression of the polypeptides relative to the analogous native polynucleotide sequences. Further, modified polypeptides may comprise mutations (single or multiple point mutations, missense mutations, nonsense mutations, deletions, insertions, etc.) relative to corresponding wild-type sequences.

The compositions of the present invention are described with reference to HIV-1 derived sequences. However, the compositions and methods of the present invention are applicable to other types of viruses as well, wherein such viruses comprise multiple subtypes, serotypes, and/or strain variations, for example, including but not limited to other non-HIV retroviruses (e.g. HTLV-1, 2), hepadnoviruses (e.g. HBV), herpesviruses (e.g. HSV-1,2, CMV, EBV, varizellazoster, etc.), flaviviruses (e.g. HCV, Yellow fever, Tick borne encephalitis, St. Louis Encephalitis, West Nile Virus, etc.), coronaviruses (e.g. SARS), paramyxoviruses (e.g., PIV, RSV, measles etc.), influenza viruses, picornaviruses, reoviruses (e.g., rotavirus), arenaviruses, rhabdoviruses, papovaviruses, parvoviruses, adenoviruses, Dengue virus, bunyaviruses (e.g. , hantavirus), calciviruses (e.g. Norwalk virus), filoviruses (e.g. , Ebola, Marburg).

### 2.3.1 MODIFICATION OF POLYNUCLEOTIDE CODING SEQUENCES

HIV-1 coding sequences, and related sequences, may be modified to have improved expression in target cells relative to the corresponding wild-type sequences. Following here are some exemplary modifications that can be made to such coding sequences.

First, the HIV-1 codon usage pattern may be modified so that the resulting nucleic acid coding sequence are comparable to codon usage found in highly expressed human genes. The HIV codon usage reflects a high content of the nucleotides A or T of the codon-triplet. The effect of the HIV-1 codon usage is a high AT content in the DNA sequence that results in a decreased translation ability and instability of the mRNA. In comparison, highly expressed human codons prefer the nucleotides G or C. The HIV coding sequences may be modified to be comparable to codon usage found in highly expressed human genes.

Second, there are inhibitory (or instability) elements (INS) located within the coding sequences of, for example, the Gag coding sequences. The RRE is a secondary RNA structure that interacts with the HIV encoded Rev-protein to overcome the expression down-regulating effects of the INS. To overcome the post-transcriptional activating mechanisms of RRE and Rev, the instability elements can be inactivated by introducing multiple point mutations that do not alter the reading frame of the encoded proteins.

Third, for some genes the coding sequence has been altered such that the polynucleotide coding sequence encodes a gene product that is inactive or non-functional (e.g., inactivated polymerase, protease, tat, rev, nef, vif, vpr, and/or vpu gene products). Example 1 describes some exemplary mutations.

The synthetic coding sequences are assembled by methods known in the art, for example by companies such as the Midland Certified Reagent Company (Midland, Texas), following the guidance of the present specification.

Some exemplary synthetic polynucleotide sequences encoding immunogenic HIV polypeptides and the polypeptides encoded thereby for use in the methods of the present invention have been described, for example, in PCT International Publication Nos. WO/00/39303, WO/00/39302, WO 00/39304, WO/02/04493, WO/03/020876, WO/03/004620, and WO/03/004657.

In a preferred embodiment, the present invention relates to polynucleotides encoding Env polypeptides and corresponding Env polypeptides. For example, the codon usage pattern for Env may be modified so that the resulting nucleic acid coding sequence is comparable to codon usage found in highly expressed human genes. Such synthetic Env sequences are capable of higher level of protein production relative to the native Env sequences (see, for example, PCT International Publication Nos. WO/00/39302). Modification of the Env polypeptide coding sequences results in improved expression relative to the wildtype coding sequences in a number of mammalian cell lines (as well as other types of cell lines, including, but not limited to, insect cells). Similar Env polypeptide coding sequences can be obtained, modified and tested for improved expression from a variety of isolates.

Further modifications of Env include, but are not limited to, generating polynucleotides that encode Env polypeptides having mutations and/or deletions therein. For instance, the hypervariable regions, V1 and/or V2, can be deleted as described herein. In addition, the variable regions V3, V4 and/ or V5 can be modified or deleted. (See e.g, US 6,602,705) Additionally, other modifications, for example to the bridging sheet region and/or to N-glycosylation sites within Env can also be performed following the teachings of the present specification. (International Publication Nos. WO/00/39303, WO/00/39302, WO 00/39304, WO/02/04493, WO/03/020876, and WO/03/004620). Other useful modifications of env are well known and include those described in Schulke et al., (J. Virol. 2002 76:7760), Yang et al. 2002, (J. Virol. 2002 76:4634), Yang et al. 2001( J. Virol. 2001 75:1165), Shu et al. (Biochem. 1999 38:5378), Farzan et al. (J.Virol. 1998 72:7620) and Xiang et al. (J.Virol. 2002 76:9888). Various combinations of these modifications can be employed to generate synthetic expression cassettes and corresponding polypeptides as described herein.

The present invention also includes expression cassettes which include synthetic sequences derived HIV genes other than Env, including but not limited to, regions within Gag, Env, Pol, as well as, tat, rev, nef, vif, vpr, and vpu. Further, the present invention includes synthetic polynucleotides and/or expression cassettes (as well as polypeptide encoded thereby) comprising two or more antigenic polypeptides. Such sequences may be used, for example, in their entirety or sequences encoding specific epitopes or antigens may be selected from the synthetic coding sequences following the teachings of the present specification and information known in the art. For example, the polypeptide sequences encoded by the polynucleotides may be subjected to computer analysis to predict antigenic peptide fragments within the full-length sequences. The corresponding polynucleotide coding fragments may then be used in the constructs of the present invention. Exemplary algorithms useful for such analysis include, but are not limited to, the following:

AMPHI. This program has been used to predict T-cell epitopes (Gao, et al., (1989) J. Immunol. 143:3007; Roberts, et al, (1996) AIDS Res Hum Retrovir 12:593; Quakyi, et al., (1992) Scand J Immunol suppl. 11:9). The AMPHI algorithm is available int the Protean package of DNASTAR, Inc. (Madison, WI, USA).

ANTIGENIC INDEX. This algorithm is useful for predicting antigenic determinants (Jameson & Wolf, (1998) CABIOS 4:181:186; Shennan, KE, et al., Hepatology 1996 Apr;23(4):688-94; Kasturi, KN, et al, J Exp Med 1995 Mar 1;181(3):1027-36; van Kampen V, et al., Mol Immunol 1994 Oct;31(15):1133-40; Ferroni P, et al., J Clin Microbiol 1993 Jun;31(6):1586-91; Beattie J, et al., Eur J Biochem 1992 Nov 15;210(1):59-66; Jones GL, et al, Mol Biochem Parasitol 1991 Sep;48(1):1-9).

HYDROPHILICITY. One algorithm useful for determining antigenic determinants from amino acid sequences was disclosed by Hopp & Woods (1981) (PNAS USA 78:3824-3828.

Default parameters, for the above-recited algorithms, may be used to determine antigenic sites. Further, the results of two or more of the above analyses may be combined to identify particularly preferred fragments.

### 2.3.2 FURTHER MODIFICATION OF POLYNUCLEOTIDE SEQUENCES AND POLYPEPTIDES ENCODED THEREBY

The immunogenic viral polypeptide-encoding expression cassettes described herein may also contain one or more further sequences encoding, for example, one or more transgenes. In one embodiment of the present invention, the polynucleotide component may comprise coding sequences for one or more HIV immunogenic polypeptides. Further, the polypeptide component may comprise one or more HIV immunogenic polypeptides.

In a different embodiment of the present invention, a polynucleotide component may comprise coding sequences for one or more HIV immunogenic polypeptides, wherein the polynucleotide component further comprises a sequence encoding an additional antigenic polypeptide, with the proviso that the additional antigenic polypeptide is not an immunogenic polypeptide derived from an HIV-1 strain. Further, the polypeptide component may comprise one or more HIV immunogenic polypeptides, wherein the polypeptide component further comprises an additional antigenic polypeptide, with the proviso that the additional antigenic polypeptide is not an immunogenic polypeptide derived from an HIV-1 strain.

Further sequences (*e*.*g*., transgenes) useful in the practice of the present invention include, but are not limited to, further sequences are those encoding further viral epitopes/antigens {including but not limited to, HCV antigens (e.g., E1, E2; Houghton, M.., et al., U.S. Patent No. 5,714,596, issued February 3, 1998; Houghton, M.., et al., U.S. Patent No. 5,712,088, issued January 27, 1998; Houghton, M.., et al., U.S. Patent No. 5,683,864, issued November 4, 1997; Weiner, A.J., et al., U.S. Patent No. 5,728,520, issued March 17, 1998; Weiner, A.J., et al., U.S. Patent No. 5,766,845, issued June 16, 1998; Weiner, A.J., et al., U.S. Patent No. 5,670,152, issued September 23, 1997), HIV antigens (e.g., derived from one or more HIV isolate); and sequences encoding tumor antigens/epitopes. Further sequences may also be derived from non-viral sources, for instance, sequences encoding cytokines such interleukin-2 (IL-2), stem cell factor (SCF), interleukin 3 (IL-3), interleukin 6 (IL-6), interleukin 12 (IL-12), G-CSF, granulocyte macrophage-colony stimulating factor (GM-CSF), interleukin-1 alpha (IL-lalpha), interleukin-11 (IL-11), MIP-1, tumor necrosis factor (TNF), leukemia inhibitory factor (LIF), c-kit ligand, thrombopoietin (TPO) and flt3 ligand, commercially available from several vendors such as, for example, Genzyme (Framingham, MA), Genentech (South San Francisco, CA), Amgen (Thousand Oaks, CA), R&D Systems and Immunex (Seattle, WA). Additional sequences are described herein below.

HIV polypeptide coding sequences can be obtained from other HIV isolates, see, e.g., Myers et al. Los Alamos Database, Los Alamos National Laboratory, Los Alamos, New Mexico (1992); Myers et al., Human Retroviruses and Aids, 1997, Los Alamos, New Mexico: Los Alamos National Laboratory. Synthetic expression cassettes can be generated using such coding sequences as starting material by following the teachings of the present specification.

Further, the synthetic expression cassettes of the present invention include related polypeptide sequences having greater than 85%, preferably greater than 90%, more preferably greater than 95%, and most preferably greater than 98% sequence identity to the polypeptides encoded by the synthetic expression cassette sequences disclosed herein.

Exemplary expression cassettes and modifications are set forth in Example 1 and are discussed further herein below.

Further, the polynucleotides of the present invention may comprise alternative polymer backbone structures such as, but not limited to, polyvinyl backbones (Pitha, Biochem Biophys Acta, 204:39,1970a; Pitha, Biopolymers, 9:965, 1970b), and morpholino backbones (Summerton, J., et al., U.S. Patent No. 5,142,047, issued 08/25/92; Summerton, J., et al., U.S. Patent No. 5,185,444 issued 02/09/93). A variety of other charged and uncharged polynucleotide analogs have been reported. Numerous backbone modifications are known in the art, including, but not limited to, uncharged linkages (e.g., methyl phosphonates, phosphotriesters, phosphoamidates, and carbamates) and charged linkages (e.g., phosphorothioates and phosphorodithioates.

### 2.3.3 EXEMPLARY CLONING VECTORS AND SYSTEMS FOR USE WITH THE POLYNUCLEOTIDE SEQUENCES ENCODING IMMUNOGENIC POLYPFPTIDES

Polynucleotide sequences for use in the gene delivery vector compositions and methods of the present invention can be obtained using recombinant methods, such as by screening cDNA and genomic libraries from cells expressing the gene, or by deriving the gene from a vector known to include the same. Furthermore, the desired gene can be isolated directly from cells and tissues containing the same, using standard techniques, such as phenol extraction and PCR of cDNA or genomic DNA. See, e.g., Sambrook et al., *supra,* for a description of techniques used to obtain and isolate DNA. The gene of interest can also be produced synthetically, rather than cloned. The nucleotide sequence can be designed with the appropriate codons for the particular amino acid sequence desired. In general, one will select preferred codons for the intended host in which the sequence will be expressed. The complete sequence is assembled from overlapping oligonucleotides prepared by standard methods and assembled into a complete coding sequence. See, e.g., Edge, Nature (1981) 292:756; Nambair et al., Science (1984) 223:1299; Jay et al., J. Biol. Chem. (1984) 259:6311; Stemmer, W.P.C., (1995) Gene 164:49-53.

Next, the gene sequence encoding the desired antigen can be inserted into a vector containing a synthetic expression cassette of the present invention. In one embodiment, polynucleotides encoding selected antigens are separately cloned into expression vectors (e.g., a first Env-coding polynucleotide in a first vector, a second analogous Env-coding polynucleotide in a second vector). In certain embodiments, the antigen is inserted into or adjacent a synthetic Gag coding sequence such that when the combined sequence is expressed it results in the production of VLPs comprising the Gag polypeptide and the antigen of interest, e.g., Env (native or modified) or other antigen(s) (native or modified) derived from HIV. Insertions can be made within the coding sequence or at either end of the coding sequence (5', amino terminus of the expressed Gag polypeptide; or 3', carboxy terminus of the expressed Gag polypeptide)(Wagner, R., et al., Arch Virol. 127:117-137, 1992; Wagner, R., et al., Virology 200:162-175, 1994; Wu, X., et al., J. Virol. 69(6):3389-3398, 1995; Wang, C-T., et al., Virology 200:524-534, 1994; Chazal, N., et al., Virology 68(1):111-122, 1994; Griffiths, J.C., et al., J. Virol. 67(6):3191-3198, 1993; Reicin, A.S., et al., J. Virol. 69(2):642-650,1995). Up to 50% of the coding sequences of p55Gag can be deleted without affecting the assembly to virus-like particles and expression efficiency (Borsetti, A., et al, J. Virol. 72(11):9313-9317, 1998; Gamier, L., et al., J Virol 72(6):4667-4677, 1998; Zhang, Y., et al., J Virol 72(3):1782-1789, 1998; Wang, C., et al., J Virol 72(10): 7950-7959, 1998). When sequences are added to the amino terminal end of Gag, the polynucleotide can contain coding sequences at the 5' end that encode a signal for addition of a myristic moiety to the Gag-containing polypeptide (e.g., sequences that encode Met-Gly).

Expression cassettes for use in the practice of the present invention can also include control elements operably linked to the coding sequence that allow for the expression of the gene *in vivo* in the subject species. For example, typical promoters for mammalian cell expression include the SV40 early promoter, a CMV promoter such as the CMV immediate early promoter, the mouse mammary tumor virus LTR promoter, the adenovirus major late promoter (Ad MLP), and the herpes simplex virus promoter, among others. Other nonviral promoters, such as a promoter derived from the murine metallothionein gene, will also find use for mammalian expression. Typically, transcription termination and polyadenylation sequences will also be present, located 3' to the translation stop codon. Preferably, a sequence for optimization of initiation of translation, located 5' to the coding sequence, is also present. Examples of transcription terminator/polyadenylation signals include those derived from SV40, as described in Sambrook et al., *supra,* as well as a bovine growth hormone terminator sequence.

Enhancer elements may also be used herein to increase expression levels of the mammalian constructs. Examples include the SV40 early gene enhancer, as described in Dijkema et al., EMBO J. (1985) 4:761, the enhancer/promoter derived from the long terminal repeat (LTR) of the Rous Sarcoma Virus, as described in Gorman et al., Proc. Natl. Acad. Sci. USA (1982b) 79:6777 and elements derived from human CMV, as described in Boshart et al., Cell (1985) 41:521, such as elements included in the CMV intron A sequence.

Furthermore, plasmids can be constructed which include a chimeric antigen-coding gene sequences, encoding, e.g., multiple antigens/epitopes of interest, for example derived from more than one viral isolate.

Typically the antigen coding sequences precede or follow the synthetic coding sequence and the chimeric transcription unit will have a single open reading frame encoding both the antigen of interest and the synthetic coding sequences. Alternatively, multi-cistronic cassettes (e.g., bi-cistronic cassettes) can be constructed allowing expression of multiple antigens from a single mRNA using the EMCV IRES, or the like.

In one embodiment of the present invention, the polynucleotide of a gene delivery vector as described herein may comprise, for example, the following:
a first expression vector comprising a first Env expression cassette, wherein the Env coding sequence is derived from a first HIV subtype, serotype, or strain, and a second expression vector comprising a second Env expression cassette, wherein the Env coding sequence is derived from a second HIV subtype, serotype, or strain. Expression cassettes comprising coding sequences of the present invention may be combined in any number of combinations depending on the coding sequence products (e.g., HIV polypeptides) to which, for example, an immunological response is desired to be raised. In yet another embodiment, synthetic coding sequences for multiple HIV-derived polypeptides may be constructed into a polycistronic message under the control of a single promoter wherein IRES are placed adjacent the coding sequence for each encoded polypeptide.

Exemplary polynucleotide sequences of interest for use in the present invention may be derived from strains including, but not limited to: subtype B-SF162, subtype C-TV1.8_2 (8_2_TV1_C.ZA), subtype C-TV1.8_5 (8_5_TV1_C.ZA), subtype C-TV2.12-5/1 (12-5_1_TV2_C.ZA), subtype C-MJ4, India subtype C-93IN101, subtype A-Q2317, subtype D-92UG001, subtype E-cm235, subtype A HIV-1 isolate Q23-17 from Kenya GenBank Accession AF004885, subtype A HIV-1 isolate 98UA0116 from Ukraine GenBank Accession AF413987, subtype A HIV-1 isolate SE8538 from Tanzania GenBank Accession AF069669, subtype A Human immunodeficiency virus 1 proviral DNA, complete genome, clone:pUG031-A1 GenBank Accession AB098330, subtype D Human immunodeficiency virus type 1 complete proviral genome, strain 92UG001 GenBank Accession AJ320484, subtype D HIV-1 isolate 94UG114 from Uganda GenBank Accession US8824, subtype D Human immunodeficiency virus type 1, isolate ELIGenBank Accession K03454, and Indian subtype C Human immunodeficiency virus type 1 subtype C genomic RNA GenBank Accession AB023804.

Polynucleotide coding sequences used in the present invention may encode functional gene products or be mutated to reduce (relative to wild-type), attenuate, inactivate, eliminate, or render non-functional the activity of the gene product(s) encoded the synthetic polynucleotide.

Once complete, the expression cassettes are typically used in constructs for nucleic acid immunization using standard gene delivery protocols. Methods for gene delivery are known in the art. See, e.g., U.S. Patent Nos. 5,399,346, 5,580,859, 5,589,466. Genes can be delivered either directly to the vertebrate subject or, alternatively, delivered *ex vivo,* to cells derived from the subject and the cells reimplanted in the subject.

In preferred embodiments, the gene delivery vectors are viral vectors. A number of viral based systems have been developed for gene transfer into mammalian cells. *See, e.g.,* WO/00/39302; WO/00/39304; WO/02/04493; WO/03/004657; WO/03/004620; and WO/03/020876; US Patent No. 6,602,705; and US Published Patent Application Nos. 20030143248 , and 20020146683 and references cited therein, for a description of various retroviral, lentiviral, pox virus, vaccinia virus, and adeno-associated viral vector systems as well as delivery of naked DNA (*e.g.*, plasmids).

In certain embodiments, the first or second gene delivery vector is an adenovirus vector. A number of adenovirus vectors have also been described. Unlike retroviruses which integrate into the host genome, adenoviruses persist extrachromosomally thus minimizing the risks associated with insertional mutagenesis (Haj-Ahmad and Graham, J. Virol. (1986) 57:267-274; Bett et al., J. Viol. (1993) 67:5911-5921; Mittereder et al., Human Gene Therapy (1994) 5:717-729; Seth et al., J. Virol. (1994) 68:933-940; Barr et al., Gene Therapy (1994) 1:51-58; Berkner, K.L. BioTechniques (1988) 6:616-629; and Rich et al., Human Gene Therapy (1993) 4:461-476).

In other embodiments, one or more of the gene delivery vectors is a bacterial vector. For example, U.S. Pat. No. 5,877,159 to Powell et al., describes live bacteria that can invade animal cells to thereby introduce a eukaryotic expression cassette encoding an antigen. In yet other embodiments, one or more of the gene delivery vectors is a fungal vector.

Molecular conjugate vectors, such as the adenovirus chimeric vectors described in Michael et al., J. Biol. Chem. (1993) 268:6866-6869 and Wagner et al., Proc. Natl. Acad. Sci. USA (1992) 89:6099-6103, can also be used for gene delivery.

Alphavirus vectors are also advantageously used in the practice of the present invention. Members of the Alphavirus genus, such as, but not limited to, vectors derived from the Sindbis, Semliki Forest, and Venezuelan Equine Encephalitis viruses, will also find use as viral vectors for delivering the polynucleotides of the present invention (for example, first and second synthetic gp140-polypeptide encoding expression cassette, wherein the first and second gp140 polypeptides are analogous and derived from different HIV subtypes, serotypes, or strains). For a description of Sindbis-virus derived vectors useful for the practice of the instant methods, see, Dubensky et al., J. Virol. (1996) 70:508-519; and International Publication Nos. WO 95/07995 and WO 96/17072; as well as, U.S. Patent No. 5,843,723 and U.S. Patent No. 5,789,245. Preferred expression systems include, but are not limited to, eucaryotic layered vector initiation systems (e.g., U.S. Patent No. 6,015,686, U.S. Patent No. 5,814,482, U.S. Patent No. 6,015,694, U.S. Patent No. 5,789,245, EP 1029068A2, International Publication No. WO 99/18226, EP 00907746A2, International Publication No. WO 97/38087). Exemplary expression systems include, but are not limited to, chimeric alphavirus replicon particles, for example, those that form VEE and SIN (see, e.g., Perri, et al., J. Virol 2003, 77(19):10394-10403; International Publication No. WO02/099035; U.S. Publication No. 20030232324). Such alphavirus-based vector systems can be used in a prime or as a boost in DNA-primed subjects or potentially as a stand-alone immunization method for the induction of neutralizing antibodies using the approaches described herein.

Gene delivery vectors may also include tissue-specific promoters to drive expression of one or more genes or sequences of interest.

Gene delivery vector constructs may be generated such that more than one gene of interest is expressed. This may be accomplished through the use of di- or oligo-cistronic cassettes (e.g., where the coding regions are separated by 80 nucleotides or less, *see generally* Levin et al., Gene 108:167-174, 1991), or through the use of Internal Ribosome Entry Sites ("IRES").

In addition, the expression cassettes of the present invention can be packaged in liposomes prior to delivery to the subject or to cells derived therefrom. Lipid encapsulation is generally accomplished using liposomes which are able to stably bind or entrap and retain nucleic acid. The ratio of condensed DNA to lipid preparation can vary but will generally be around 1:1 (mg DNA:micromoles lipid), or more of lipid. For a review of the use of liposomes as carriers for delivery of nucleic acids, see, Hug and Sleight, Biochim. Biophys. Acta. (1991) 1097:1-17; Straubinger et al., in Methods of Enzymology (1983), Vol. 101, pp. 512-527.

Liposomal preparations for use in the present invention include cationic (positively charged), anionic (negatively charged) and neutral preparations, with cationic liposomes particularly preferred. Cationic liposomes have been shown to mediate intracellular delivery of plasmid DNA (Felgner et al., Proc. Natl. Acad. Sci. USA (1987) 84:7413-7416); mRNA (Malone et al., Proc. Natl. Acad. Sci. USA (1989) 86:6077-6081); and purified transcription factors (Debs et al., J. Biol. Chem. (1990) 265:10189-10192), in functional form.

Cationic liposomes are readily available. For example, N[1-2,3-dioleyloxy)propyl]-N,N,N-triethylammonium (DOTMA) liposomes are available under the trademark Lipofectin, from GIBCO BRL, Grand Island, NY. (See, also, Felgner et al., Proc. Natl. Acad. Sci. USA (1987) 84:7413-7416). Other commercially available lipids include (DDAB/DOPE) and DOTAP/DOPE (Boerhinger). Other cationic liposomes can be prepared from readily available materials using techniques well known in the art. See, e.g., Szoka et al., Proc. Natl. Acad. Sci. USA (1978) 75:4194-4198; International Publication No. WO 90/11092 for a description of the synthesis of DOTAP (1,2-bis(oleoyloxy)-3-(trimethylammonio)propane) liposomes.

Similarly, anionic and neutral liposomes are readily available, such as, from Avanti Polar Lipids (Birmingham, AL), or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art.

The liposomes can comprise multilammelar vesicles (MLVs), small unilamellar vesicles (SUVs), or large unilamellar vesicles (LUVs). The various liposome-nucleic acid complexes are prepared using methods known in the art. See, e.g., Straubinger et al., in METHODS OF IMMUNOLOGY (1983), Vol. 101, pp. 512-527; Szoka et al., Proc. Natl. Acad. Sci. USA (1978) 75:4194-4198; Papahadjopoulos et al., Biochim. Biophys. Acta (1975) 394:483; Wilson et al., Cell (1979) 17:77); Deamer and Bangham, Biochim. Biophys. Acta (1976) 443:629; Ostro et al., Biochem. Biophys. Res. Commun. (1977) 76:836; Fraley et al., Proc. Natl. Acad. Sci. USA (1979) 76:3348); Enoch and Strittmatter, Proc. Natl. Acad. Sci. USA (1979) 76:145); Fraley et al., J. Biol. Chem. (1980) 255:10431; Szoka and Papahadjopoulos, Proc. Natl. Acad. Sci. USA (1978) 75:145; and Schaefer-Ridder et al., Science (1982) 215:166.

The DNA and/or protein antigen(s) can also be delivered in cochleate lipid compositions similar to those described by Papahadjopoulos et al., Biochem. Biophys. Acta. (1975) 394:483-491. See, also, U.S. Patent Nos. 4,663,161 and 4,871,488.

The expression cassettes of interest may also be encapsulated, adsorbed to, or associated with, particulate carriers. Such carriers present multiple copies of a selected antigen to the immune system and promote trapping and retention of antigens in local lymph nodes. The particles can be phagocytosed by macrophages and can enhance antigen presentation through cytokine release. Examples of particulate carriers include those derived from polymethyl methacrylate polymers, as well as microparticles derived from poly(lactides) and poly(lactide-co-glycolides), known as PLG. See, e.g., Jeffery et al., Pharm. Res. (1993) 10:362-368; McGee JP, et al., J Microencapsul. 14(2):197-210, 1997; O'Hagan DT, et al., Vaccine 11(2):149-54, 1993. Suitable microparticles may also be manufactured in the presence of charged detergents, such as anionic or cationic detergents, to yield microparticles with a surface having a net negative or a net positive charge. For example, microparticles manufactured with anionic detergents, such as hexadecyltrimethylammonium bromide (CTAB), i.e. CTAB-PLG microparticles, adsorb negatively charged macromolecules, such as DNA. (see, e.g., International Publication No. WO 00/06123).

Furthermore, other particulate systems and polymers can be used for the *in vivo* or *ex vivo* delivery of the gene of interest. For example, polymers such as polylysine, polyarginine, polyornithine, spermine, spermidine, as well as conjugates of these molecules, are useful for transferring a nucleic acid of interest. Similarly, DEAE dextran-mediated transfection, calcium phosphate precipitation or precipitation using other insoluble inorganic salts, such as strontium phosphate, aluminum silicates including bentonite and kaolin, chromic oxide, magnesium silicate, talc, and the like, will find use with the present methods. See, e.g., Felgner, P.L., Advanced Drug Delivery Reviews (1990) 5:163-187, for a review of delivery systems useful for gene transfer. Peptoids (Zuckerman, R.N., et al., U.S. Patent No. 5,831,005) may also be used for delivery of a construct of the present invention.

In some embodiments of the present invention, alum and PLG are useful delivery adjuvants that enhance immunity to polynucleotide vaccines (e.g., DNA vaccines). Further embodiments include, but are not limited to, toxoids, cytokines, and co-stimulatory molecules may also be used as genetic adjuvants with polynucleotide vaccines.

Gene delivery vectors carrying a synthetic expression cassette of the present invention are formulated into compositions for delivery to the vertebrate subject. These compositions may either be prophylactic (to prevent infection) or therapeutic (to treat disease after infection). If prevention of disease is desired, the compositions are generally administered prior to primary infection with the pathogen of interest. If treatment is desired, e.g., the reduction of symptoms or recurrences, the compositions are generally administered subsequent to primary infection. The compositions will comprise a "therapeutically effective amount" of the gene of interest such that an amount of the antigen can be *produced in vivo* so that an immune response is generated in the individual to which it is administered. The exact amount necessary will vary depending on the subject being treated; the age and general condition of the subject to be treated; the capacity of the subject's immune system to synthesize antibodies; the degree of protection desired; the severity of the condition being treated; the particular antigen selected and its mode of administration, among other factors. An appropriate effective amount can be readily determined by one of skill in the art. Thus, a "therapeutically effective amount" will fall in a relatively broad range that can be determined through routine trials.

The compositions will generally include one or more "pharmaceutically acceptable excipients or vehicles" such as water, saline, glycerol, polyethyleneglycol, hyaluronic acid, ethanol, etc. Additionally, auxiliary substances, such as wetting or emulsifying agents, pH buffering substances, and the like, may be present in such vehicles. Certain facilitators of nucleic acid uptake and/or expression can also be included in the compositions or coadministered, such as, but not limited to, bupivacaine, cardiotoxin and sucrose.

Once formulated, the compositions of the invention can be administered directly to the subject (e.g., as described above) or, alternatively, delivered *ex vivo,* to cells derived from the subject, using methods such as those described above. For example, methods for the *ex vivo* delivery and reimplantation of transformed cells into a subject are known in the art and can include, e.g., dextran-mediated transfection, calcium phosphate precipitation, polybrene mediated transfection, lipofectamine and LT-1 mediated transfection, protoplast fusion, electroporation, encapsulation of the polynucleotide(s) (with or without the corresponding antigen) in liposomes, and direct microinjection of the DNA into nuclei.

The gene delivery vectors can be administered *in vivo* in a variety of ways. The vectors can be injected either subcutaneously, epidermally, intradermally, intramucosally such as nasally, rectally and vaginally, intraperitoneally, intravenously, orally or intramuscularly. Delivery into cells of the epidermis is particularly preferred as this mode of administration provides access to skin-associated lymphoid cells and provides for a transient presence of DNA in the recipient. Other modes of administration include oral and pulmonary administration, suppositories, needle-less injection, transcutaneous and transdermal applications. Dosage treatment may be a single dose schedule or a multiple dose schedule. Administration of polypeptides encoding immunogenic polypeptides is combined with administration of analogous immunogenic polypeptides following the methods of the present invention.

### 2.3.4 EXPRESSION OF SYNTHETIC SEQUENCES ENCODING HIV-1 POLYPEPTIDES AND RELATED POLYPEPTIDES

Immunogenic viral polypeptide-encoding sequences of the present invention can be cloned into a number of different expression vectors/host cell systems to provide immunogenic polypeptides for the polypeptide component of the immune-response generating compositions of the present invention. For example, DNA fragments encoding HIV polypeptides can be cloned into eucaryotic expression vectors, including, a transient expression vector, CMV-promoter-based mammalian vectors, and a shuttle vector for use in baculovirus expression systems. Synthetic polynucleotide sequences (e.g., codon optimized polynucleotide sequences) and wild-type sequences can typically be cloned into the same vectors. Numerous cloning vectors are known to those of skill in the art, and the selection of an appropriate cloning vector is a matter of choice. See, generally, Sambrook et al, *supra.* The vector is then used to transform an appropriate host cell. Suitable recombinant expression systems include, but are not limited to, bacterial, mammalian, baculovirus/insect, vaccinia, Semliki Forest virus (SFV), Alphaviruses (such as, Sindbis, Venezuelan Equine Encephalitis (VEE)), mammalian, yeast and Xenopus expression systems, well known in the art. Particularly preferred expression systems are mammalian cell lines, vaccinia, Sindbis, eucaryotic layered vector initiation systems (e.g., US Patent No. 6,015,686, US Patent No. 5, 814,482, US Patent No. 6,015,694, US Patent No. 5,789,245, EP 1029068A2, PCT International Publication No. WO 9918226A2/A3, EP 00907746A2, PCT International Publication No. WO 9738087A2), insect and yeast systems.

A number of host cells for such expression systems are also known in the art. For example, mammalian cell lines are known in the art and include immortalized cell lines available from the American Type Culture Collection (A.T.C.C.), such as, but not limited to, Chinese hamster ovary (CHO) cells, HeLa cells, baby hamster kidney (BHK) cells, monkey kidney cells (COS), as well as others. Similarly, bacterial hosts such as *E. coli, Bacillus subtilis,* and *Streptococcus spp*., will find use with the present expression constructs. Yeast hosts useful in the present invention include *inter alia, Saccharomyces cerevisiae, Candida albicans, Candida maltosa, Hansenula polymorpha, Kluyveromyces fragilis, Kluyveromyces lactis, Pichia guillerimondii, Pichia pastoris, Schizosaccharomyces pombe* and *Yarrowia lipolytica.* Insect cells for use with baculovirus expression vectors include, *inter alia, Aedes aegypti, Autographa californica, Bombyx mori, Drosophila melanogaster, Spodoptera frugiperda,* and *Trichoplusia ni.* See, e.g., Summers and Smith, *Texas Agricultural Experiment Station Bulletin No. 1555* (1987).

Viral vectors can be used for expression of polypeptides in eucaryotic cells, such as those derived from the pox family of viruses, including vaccinia virus and avian poxvirus. For example, a vaccinia based infection/transfection system, as described in Tomei et al., J. Viol. (1993) 67:4017-4026 and Selby et al., J. Gen. Virol. (1993) 74:1103-1113, will also find use with the present invention. A vaccinia based infection/transfection system can be conveniently used to provide for inducible, transient expression of the coding sequences of interest in a host cell. In this system, cells are first infected *in vitro* with a vaccinia virus recombinant that encodes the bacteriophage T7 RNA polymerase. This polymerase displays exquisite specificity in that it only transcribes templates bearing T7 promoters. Following infection, cells are transfected with the polynucleotide of interest, driven by a T7 promoter. The polymerase expressed in the cytoplasm from the vaccinia virus recombinant transcribes the transfected DNA into RNA that is then translated into protein by the host translational machinery. The method provides for high level, transient, cytoplasmic production of large quantities of RNA and its translation products. See, e.g., Elroy-Stein and Moss, Proc. Natl. Acad. Sci. USA (1990) 87:6743-6747; Fuerst et al., Proc. Natl. Acad. Sci. USA (1986) 83:8122-8126.

As an alternative approach to infection with vaccinia or avipox virus recombinants, an amplification system can be used that will lead to high level expression following introduction into host cells. Specifically, a T7 RNA polymerase promoter preceding the coding region for T7 RNA polymerase can be engineered. Translation of RNA derived from this template will generate T7 RNA polymerase which in turn will transcribe more template. Concomitantly, there will be a cDNA whose expression is under the control of the T7 promoter. Thus, some of the T7 RNA polymerase generated from translation of the amplification template RNA will lead to transcription of the desired gene. Because some T7 RNA polymerase is required to initiate the amplification, T7 RNA polymerase can be introduced into cells along with the template(s) to prime the transcription reaction. The polymerase can be introduced as a protein or on a plasmid encoding the RNA polymerase. For a further discussion of T7 systems and their use for transforming cells, see, e.g., PCT International Publication No. WO 94/26911; Studier and Moffatt, J. Mol. Biol. (1986) 189:113-130; Deng and Wolff, Gene (1994) 143:245-249; Gao et al., Biochem. Biophys. Res. Commun. (1994) 200:1201-1206; Gao and Huang, Nuc. Acids Res. (1993) 21:2867-2872; Chen et al., Nuc. Acids Res. (1994) 22:2114-2120; and U.S. Patent No. 5,135,855.

These vectors are transfected into an appropriate host cell. The cell lines are then cultured under appropriate conditions and the levels of any appropriate polypeptide product can be evaluated in supernatants. For example, p24 can be used to evaluate Gag expression; gp160, gp140 or gp120 can be used to evaluate Env expression; p6pol can be used to evaluate Pol expression; prot can be used to evaluate protease; p15 for RNAseH; p31 for Integrase; and other appropriate polypeptides for Vif, Vpr, Tat, Rev, Vpu and Nef.

Further, modified polypeptides can also be used, for example, other Env polypeptides include, but are not limited to, for example, native gp160, oligomeric gp140, monomeric gp120 as well as modified and/or synthetic sequences of these polypeptides.

Western Blot analysis can be used to show that cells containing the synthetic expression cassette produce the expected protein, typically at higher percell concentrations than cells containing the native expression cassette. The HIV proteins can be seen in both cell lysates and supernatants.

Fractionation of the supernatants from mammalian cells transfected with the synthetic expression cassette can be used to show that the cassettes provide superior production of HIV proteins and relative to the wild-type sequences.

Efficient expression of these HIV-containing polypeptides in mammalian cell lines provides the following benefits: the polypeptides are free of baculovirus contaminants; production by established methods approved by the FDA; increased purity; greater yields (relative to native coding sequences); and a novel method of producing the Sub HIV-containing polypeptides in CHO cells which is not feasible in the absence of the increased expression obtained using the constructs of the present invention. Exemplary Mammalian cell lines include, but are not limited to, BHK, VERO, HT1080, 293, 293T, RD, COS-7, CHO, Jurkat, HUT, SUPT, C8166, MOLT4/clone8, MT-2, MT-4, H9, PM1, CEM, and CEMX174 (such cell lines are available, for example, from the A.T.C.C.).

The desired polypeptide encoding sequences can be cloned into any number of commercially available vectors to generate expression of the polypeptide in an appropriate host system. These systems include, but are not limited to, the following: baculovirus expression {Reilly, P.R., et al., BACULOVIRUS EXPRESSION VECTORS: A LABORATORY MANUAL (1992); Beames, et al., Biotechniques 11:378 (1991); Pharmingen; Clontech, Palo Alto, CA)}, vaccinia expression {Earl, P. L., et al., "Expression of proteins in mammalian cells using vaccinia" In Current Protocols in Molecular Biology (F. M. Ausubel, et al. Eds.), Greene Publishing Associates & Wiley Interscience, New York (1991); Moss, B., et al., U.S. Patent Number 5,135,855, issued 4 August 1992}, expression in bacteria {Ausubel, F.M., et al., CURRENT PROTOCOLS IN MOLECULAR BIOLOGY, John Wiley and Sons, Inc., Media PA; Clontech}, expression in yeast {Rosenberg, S. and Tekamp-Olson, P., U.S. Patent No. RE35,749, issued, March 17, 1998; Shuster, J.R., U.S. Patent No. 5,629,203, issued May 13, 1997; Gellissen, G., et al., Antonie Van Leeuwenhoek, 62(1-2):79-93 (1992); Romanos, M.A., et al., Yeast 8(6):423-488 (1992); Goeddel, D.V., Methods in Enzymology 185 (1990); Guthrie, C., and G.R. Fink, Methods in Enzymology 194 (1991)}, expression in mammalian cells {Clontech; Gibco-BRL, Ground Island, NY; *e.g.*, Chinese hamster ovary (CHO) cell lines (Haynes, J., et al., Nuc. Acid. Res. 11:687-706 (1983);1983, Lau, Y.F., et al., Mol. Cell. Biol. 4:1469-1475 (1984); Kaufman, R. J., "Selection and coamplification of heterologous genes in mammalian cells," in Methods in Enzymology, vol. 185, pp537-566. Academic Press, Inc., San Diego CA (1991)}, and expression in plant cells {plant cloning vectors, Clontech Laboratories, Inc., Palo Alto, CA, and Pharmacia LKB Biotechnology, Inc., Pistcataway, NJ; Hood, E., et al., J. Bacteriol. 168:1291-1301 (1986); Nagel, R., et al., FEMS Microbiol. Lett. 67:325 (1990); An, et al., "Binary Vectors", and others in Plant Molecular Biology Manual A3:1-19 (1988); Miki, B.L.A., et al., pp.249-265, and others in Plant DNA Infectious Agents (Hohn, T., et al., eds.) Springer-Verlag, Wien, Austria, (1987); Plant Molecular Biology: Essential Techniques, P.G. Jones and J.M. Sutton, New York, J. Wiley, 1997; Miglani, Gurbachan Dictionary of Plant Genetics and Molecular Biology, New York, Food Products Press, 1998; Henry, R. J., Practical Applications of Plant Molecular Biology, New York, Chapman & Hall, 1997}.

In addition to the mammalian, insect, and yeast vectors, the synthetic expression cassettes of the present invention can be incorporated into a variety of expression vectors using selected expression control elements. Appropriate vectors and control elements for any given cell can be selected by one having ordinary skill in the art in view of the teachings of the present specification and information known in the art about expression vectors.

For example, a synthetic coding sequence can be inserted into a vector that includes control elements operably linked to the desired coding sequence, which allow for the expression of the coding sequence in a selected celltype. For example, typical promoters for mammalian cell expression include the SV40 early promoter, a CMV promoter such as the CMV immediate early promoter (a CMV promoter can include intron A), RSV, HIV-Ltr, the mouse mammary tumor virus LTR promoter (MMLV-ltr), the adenovirus major late promoter (Ad MLP), and the herpes simplex virus promoter, among others. Other nonviral promoters, such as a promoter derived from the murine metallothionein gene, will also find use for mammalian expression. Typically, transcription termination and polyadenylation sequences will also be present, located 3' to the translation stop codon. Preferably, a sequence for optimization of initiation of translation, located 5' to the coding sequence, is also present. Examples of transcription terminator/polyadenylation signals include those derived from SV40, as described in Sambrook, et al., *supra,* as well as a bovine growth hormone terminator sequence. Introns, containing splice donor and acceptor sites, may also be designed into the constructs for use with the present invention (Chapman et al., Nuc. Acids Res. (1991) 19:3979-3986).

Enhancer elements may also be used herein to increase expression levels of the mammalian constructs. Examples include the SV40 early gene enhancer, as described in Dijkema et al., EMBO J. (1985) 4:761, the enhancer/promoter derived from the long terminal repeat (LTR) of the Rous Sarcoma Virus, as described in German et al., Proc. Natl. Acad. Sci. USA (1982b) 79:6777 and elements derived from human GMV, as described in Boshart et al., Cell (1985) 41:521, such as elements included in the CMV intron A sequence (Chapman et al., Nuc. Acids Res. (1991) 19:3979-3986).

Also included in the invention are expression cassettes, comprising coding sequences and expression control elements that allow expression of the coding regions in a suitable host. The control elements generally include a promoter, translation initiation codon, and translation and transcription termination sequences, and an insertion site for introducing the insert into the vector. Translational control elements useful in expression of the polypeptides of the present invention have been reviewed by M. Kozak (e.g., Kozak, M., Mamm. Genome 7(8):563-574, 1996; Kozak, M., Biochimie 76(9):815-821, 1994; Kozak, M., J Cell Biol 108(2):229-241, 1989; Kozak, M., and Shatkin, A.J., Methods Enzymol 60:360-375, 1979).

Expression in yeast systems has the advantage of commercial production. Recombinant protein production by vaccinia and CHO cell lines have the advantage of being mammalian expression systems. Further, vaccinia virus expression has several advantages including the following: (i) its wide host range; (ii) faithful post-transcriptional modification, processing, folding, transport, secretion, and assembly of recombinant proteins; (iii) high level expression of relatively soluble recombinant proteins; and (iv) a large capacity to accommodate foreign DNA.

The recombinantly expressed polypeptides from immunogenic HIV polypeptide-encoding expression cassettes are typically isolated from lysed cells or culture media. Purification can be carried out by methods known in the art including salt fractionation, ion exchange chromatography, gel filtration, size-exclusion chromatography, size-fractionation, and affinity chromatography. Immunoaffinity chromatography can be employed using antibodies generated based on, for example, HIV antigens. Isolation of oligomeric forms of HIV envelope protein has been previously described (see, e.g., PCT International Application No. WO100/39302).

Advantages of expressing the proteins of the present invention using mammalian cells include, but are not limited to, the following: well-established protocols for scale-up production; cell lines are suitable to meet good manufacturing process (GMP) standards; culture conditions for mammalian cells are known in the art.

### 2.3.5 IMMUNOGENICITY ENHANCING COMPONENTS FOR USE WITH THE POLYPEPTIDE COMPONENT OF THE PRESENT INVENTION

Compositions of the present invention for generating an immune response in a mammal, for example, comprising first and second gene delivery vectors can include various excipients, adjuvants, carriers, auxiliary substances, modulating agents, and the like. An appropriate effective amount can be determined by one of skill in the art.

The optional polypeptide component may comprise a carrier wherein the carrier is a molecule that does not itself induce the production of antibodies harmful to the individual receiving the composition. Suitable carriers are typically large, slowly metabolized macromolecules such as proteins, polysaccharides, polylactic acids, polyglycollic acids, polymeric amino acids, amino acid copolymers, lipid aggregates (such as oil droplets or liposomes), and inactive virus particles. Examples of particulate carriers include those derived from polymethyl methacrylate polymers, as well as microparticles derived from poly(lactides) and poly(lactide-co-glycolides), known as PLG. See, e.g., Jeffery et al., Pharm. Res. (1993) 10:362-368; McGee JP, et al., J Microencapsul. 14(2):197-210, 1997; O'Hagan DT, et al., Vaccine 11(2):149-54, 1993. Such carriers are well known to those of ordinary skill in the art. Additionally, these carriers may function as immunostimulating agents ("adjuvants"). Furthermore, the antigen may be conjugated to a bacterial toxoid, such as toxoid from diphtheria, tetanus, cholera, etc., as well as toxins derived from *E. coli.*

Adjuvants may also be used to enhance the effectiveness of the compositions. Such adjuvants include, but are not limited to: (1) aluminum salts (alum), such as aluminum hydroxide, aluminum phosphate, aluminum sulfate, etc.; (2) oil-in-water emulsion formulations (with or without other specific immunostimulating agents such as muramyl peptides (see below) or bacterial cell wall components), such as for example (a) MF59 (PCT International Publication No. WO 90/14837), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (3) saponin adjuvants, such as Stimulon™ (Cambridge Bioscience, Worcester, MA) may be used or particle generated therefrom such as ISCOMs (immunostimulating complexes); (4) Complete Freunds Adjuvant (CFA) and Incomplete Freunds Adjuvant (IFA); (5) cytokines, such as interleukins (IL-1, IL-2, etc.), macrophage colony stimulating factor (M-CSF), tumor necrosis factor (TNF), etc.; (6) oligonucleotides or polymeric molecules encoding immunostimulatory CpG motifs (Davis, H.L., et al., J. Immunology 160:870-876, 1998; Sato, Y. et al., Science 273:352-354, 1996) or complexes of antigens/oligonucleotides {Polymeric molecules include double and single stranded RNA and DNA, and backbone modifications thereof, for example, methylphosphonate linkages; or (7) detoxified mutants of a bacterial ADP-ribosylating toxin such as a cholera toxin (CT), a pertussis toxin (PT), or an *E. coli* heat-labile toxin (LT), particularly LT-K63 (where lysine is substituted for the wild-type amino acid at position 63) LT-R72 (where arginine is substituted for the wild-type amino acid at position 72), CT-S109 (where serine is substituted for the wild-type amino acid at position 109), and PT-K9/G129 (where lysine is substituted for the wild-type amino acid at position 9 and glycine substituted at position 129) (see, e.g., PCT International Publication Nos. WO/93/13202 and WO/92/19265); (8) Muramyl peptides include, but are not limited to, N-acetyl-muramyl-L-threonyl-D-isoglutamine (thr-MDP), N-acteyl-normuramyl-L-alanyl-D-isogluatme (nor-MDP), N-acetylmuramyl-L-alanyl-D-isogluatminyl-L-alanine-2-(1'-2'-dipalmitoyl-*sn*-glycero-3-huydroxyphosphoryloxy)-ethylamine (MTP-PE), etc.; (9) Iscomatrix (CSL Limited,Victoria, Australia; also, see, e.g., Morein B, Bengtsson KL, "Immunomodulation by iscoms, immune stimulating complexes," Methods. Sep;19(1):94-102, 1999) and (10) other substances that act as immunostimulating agents to enhance the effectiveness of the composition (e.g., Alum and CpG oligonucleotides).

Preferred adjuvants include, but are not limited to, MF59 and Iscomatrix.

Dosage treatment with the optional polypeptide component of the immune stimulating compositions of the present invention may be a single dose schedule or a multiple dose schedule. A multiple dose schedule is one in which a primary course of vaccination may be with 1-10 separate doses, followed by other doses given at subsequent time intervals, chosen to maintain and/or reinforce the immune response, for example at 1-4 months for a second dose, and if needed, a subsequent dose(s) after several months. The dosage regimen will also, at least in part, be determined by the need of the subject and be dependent on the judgment of the practitioner.

Direct delivery of the optional polypeptide component of the immune-response generating compositions of the present invention is generally accomplished, with or without adjuvants, by injection using either a conventional syringe or a gene gun, such as the Accell® gene delivery system (Chiron Corporation, Oxford, England). The polypeptides can be injected either subcutaneously, epidermally, intradermally, intramucosally such as nasally, rectally and vaginally, intraperitoneally, intravenously, orally or intramuscularly. Other modes of administration include oral and pulmonary administration, suppositories, and needle-less injection. Dosage treatment may be a single dose schedule or a multiple dose schedule. Administration of polypeptides may also be combined with administration of adjuvants or other substances.

### 2.3.6 IMMUNOMODULATORY MOLECULES

In some embodiments of the present invention, one or more of the gene delivery vectors can be constructed to encode a cytokine or other immunomodulatory molecule. For example, nucleic acid sequences encoding native IL-2 and gamma-interferon can be obtained as described in US Patent Nos. 4,738,927 and 5,326,859, respectively, while useful muteins of these proteins can be obtained as described in U.S. Patent No. 4,853,332. Nucleic acid sequences encoding the short and long forms of mCSF can be obtained as described in US Patent Nos. 4,847,201 and 4,879,227, respectively. In particular aspects of the invention, retroviral vectors expressing cytokine or immunomodulatory genes can be produced (e.g., PCT International Publication No. WO/94/02951).

Examples of suitable immunomodulatory molecules for use herein include the following: IL-1 and IL-2 (Karupiah et al. (1990) J. Immunology 144:290-298, Weber et al. (1987) J. Exp. Med. 166:1716-1733, Gansbacher et al. (1990) J. Exp. Med. 172:1217-1224, and U.S. Patent No. 4,738,927); IL-3 and IL-4 (Tepper et al. (1989) Cell 57:503-512, Golumbek et al. (1991) Science 254:713-716, and U.S. Patent No. 5,017,691); IL-5 and IL-6 (Brakenhof et al. (1987) J. Immunol. 139:4116-4121, and PCT International Publication No. WO 90/06370); IL-7 (U.S. Patent No. 4,965,195); IL-8, IL-9, IL-10, IL-11, IL-12, and IL-13 (*Cytokine Bulletin,* Summer 1994); IL- 14 and IL-15; alpha interferon (Finter et al. (1991) Drugs 42:749-765, U.S. Patent Nos. 4,892,743 and 4,966,843, PCT International Publication No. WO 85/02862, Nagata et al. (1980) Nature 284:316-320, Familletti et al. (1981) Methods in Enz. 78:387-394, Twu et al. (1989) Proc. Natl. Acad. Sci. USA 86:2046-2050, and Faktor et al. (1990) Oncogene 5:867-872); beta-interferon (Seif et al. (1991) J. Virol. 65:664-671); gamma-interferons (Radford et al. (1991) The American Society of Hepatology 20082015, Watanabe et al. (1989) Proc. Natl. Acad. Sci. USA 86:9456-9460, Gansbacher et al. (1990) Cancer Research 50:7820-7825, Maio et al. (1989) Can. Immunol. Immunother. 30:34-42, and U.S. Patent Nos. 4,762,791 and 4,727,138); G-CSF (U.S. Patent Nos. 4,999,291 and 4,810,643); GM-CSF (PCT International Publication No. WO 85/04188).

Immunomodulatory factors may also be agonists, antagonists, or ligands for these molecules. For example, soluble forms of receptors can often behave as antagonists for these types of factors, as can mutated forms of the factors themselves.

Nucleic acid molecules that encode the above-described substances, as well as other nucleic acid molecules that are advantageous for use within the present invention, may be readily obtained from a variety of sources, including, for example, depositories such as the American Type Culture Collection, or from commercial sources such as British Bio-Technology Limited (Cowley, Oxford England). Representative examples include BBG 12 (containing the GM-CSF gene coding for the mature protein of 127 amino acids), BBG 6 (which contains sequences encoding gamma interferon), A.T.C.C. Deposit No. 39656 (which contains sequences encoding TNF), A.T.C.C. Deposit No. 20663 (which contains sequences encoding alpha-interferon), A.T.C.C. Deposit Nos. 31902, 31902 and 39517 (which contain sequences encoding beta-interferon), A.T.C.C. Deposit No. 67024 (which contains a sequence which encodes Interleukin-1b), A.T.C.C. Deposit Nos. 39405, 39452, 39516, 39626 and 39673 (which contain sequences encoding Interleukin-2), A.T.C.C. Deposit Nos. 59399, 59398, and 67326 (which contain sequences encoding Interleukin-3), A.T.C.C. Deposit No. 57592 (which contains sequences encoding Interleukin-4), A.T.C.C. Deposit Nos. 59394 and 59395 (which contain sequences encoding Interleukin-5), and A.T.C.C. Deposit No. 67153 (which contains sequences encoding Interleukin-6).

Plasmids containing cytokine genes or immunomodulatory genes (PCT International Publication Nos. WO 94/02951 and WO 96/21015) can be digested with appropriate restriction enzymes, and DNA fragments containing the particular gene of interest can be inserted into a gene transfer vector using standard molecular biology techniques. (*See, e.g.,* Sambrook et al., *supra.,* or Ausubel et al, (eds) Current Protocols in Molecular Biology, Greene Publishing and Wiley-Interscience).

Polynucleotide sequences coding for the above-described molecules can be obtained using recombinant methods, such as by screening cDNA and genomic libraries from cells expressing the gene, or by deriving the gene from a vector known to include the same. For example, plasmids that contain sequences that encode altered cellular products may be obtained from a depository such as the A.T.C.C., or from commercial sources. Plasmids containing the nucleotide sequences of interest can be digested with appropriate restriction enzymes, and DNA fragments containing the nucleotide sequences can be inserted into a gene transfer vector using standard molecular biology techniques.

Alternatively, cDNA sequences for use with the present invention may be obtained from cells that express or contain the sequences, using standard techniques, such as phenol extraction and PCR of cDNA or genomic DNA. See, e.g., Sambrook et al., *supra,* for a description of techniques used to obtain and isolate DNA. Briefly, mRNA from a cell which expresses the gene of interest can be reverse transcribed with reverse transcriptase using oligo-dT or random primers. The single stranded cDNA may then be amplified by PCR (see U.S. Patent Nos. 4,683,202, 4,683,195 and 4,800,159, see also PCR Technology: Principles and Applications for DNA Amplification, Erlich (ed.), Stockton Press, 1989)) using oligonucleotide primers complementary to sequences on either side of desired sequences.

The nucleotide sequence of interest can also be produced synthetically, rather than cloned, using a DNA synthesizer (*e.g*., an Applied Biosystems Model 392 DNA Synthesizer, available from ABI, Foster City, California). The nucleotide sequence can be designed with the appropriate codons for the expression product desired. The complete sequence is assembled from overlapping oligonucleotides prepared by standard methods and assembled into a complete coding sequence. See, e.g., Edge (1981) Nature 292:756; Nambair et al. (1984) Science 223:1299; Jay et al. (1984) J. Biol. Chem. 259:6311.

### 2.4.0 GENERATION OF IMMUNE RESPONSE IN TREATED SUBJECTS

As noted above, the gene delivery vectors described herein can be used to generate an immune response in a subject, for example, by administering first and second gene delivery vectors of the present invention (see, Table 3).

### 3.0.0 APPLICATIONS OF THE PRESENT INVENTION TO HIV

While not desiring to be bound by any particular model, theory, or hypothesis, the following information is presented to provide a more complete understanding of the present invention.

Protection against HIV infection will likely require potent and broadly reactive pre-existing neutralizing antibodies in vaccinated individuals exposed to a virus challenge. Although cellular immune responses are desirable to control viremia in those who get infected, protection against infection has not been demonstrated for vaccine approaches that rely exclusively on the induction of these responses. For this reason, experiments performed in support of the present invention used combination prime-boost approaches that employ polynucleotide components and optionally a polypeptide component, wherein the polynucleotide components encode, for example, analogous V-deleted envelope antigens from primary HIV isolates (e.g., R5 subtype B (HIV-1_{SF162}) and subtype C (HIV-1_{TVI}) strains), and the polypeptide component comprises at least one of these antigens.

The gene delivery vectors of the present invention preferably comprise adenovirus-based vectors and alphavirus replicons. Efficient in vivo expression of sequences in such vectors has been described. The optional polypeptide component of the present invention may be administered, for example, by booster immunizations with HIV (*e.g.*, Env) proteins in MF59 or Iscomatrix adjuvant.

All protein preparations are highly purified and extensively characterized by biophysical and immunochemical methodologies. Although any HIV viral protein may also be employed in the practice of the present invention, in a preferred embodiment V1-, V2-, and/or V3-modified/deleted envelope DNA and corresponding polypeptides are good candidates for use in the compositions of the present invention.

One embodiment of this aspect of the present invention may be described generally as follows. Antigens are selected for the vaccine composition(s). Polynucleotides encoding Env polypeptides and Env polypeptides are typically employed in a composition for generating an immune response comprising a polynucleotide component and a polypeptide component.

Some factors that may be considered in HIV envelope vaccine design are as follows. A fundamental criterion of an effective HIV vaccine is its ability to induce broad and potent neutralizing antibody responses against prevalent HIV strains. The important contribution of neutralizing antibodies in preventing the establishment of HIV, SIV and SHIV infection or delaying the onset of disease is highlighted by several studies. First, the emergence of neutralizationresistant viruses coincides or precedes the development of disease in infected animals (Burns (1993) J Virol, 67:4104-13; Cheng-Mayer et al. (1999) J. Virol. 73:5294-5300; Narayan et al. (1999) Virology 256:54-63). Second, the preinfusion of high concentrations of potent neutralizing monoclonal antibodies (mAbs) in the blood circulation of macaques, chimpanzees and SCID mice prior to their challenge with HIV, SIV or SHIV viruses, offers protection or delays the onset of disease (Conley et al. (1996) J. Virol. 70:6751-6758; Emini et al. (1992) Nature (London) 355:728-730; Gauduin et al. (1997) Nat Med. 3:1389-93; Mascola et al. (1999) J virol. 73:4009-18; Mascola et al. (2000) Nature Med. 6(2):207-210; Baba et al. (2000) Nature Med. 6(2):200-206). Similarly, infusion of neutralizing antibodies collected from the serum of HIV-1-infected chimpanzees to naïve pig-tailed macaques protects the latter animals from subsequent viral challenge by SHIV viruses (Shibata et al (1999) Nature Medicine 5:204-210). Moreover, envelope-based vaccines have demonstrated protection against infection in non-human primate models. Vaccines that exclude Env-polypeptides generally confer less protective efficacy (see, e.g., Hu, S.L., et al., Recombinant subunit vaccines as an approach to study correlates of protection against primate lentivirus infection, Immunol Lett. Jun;51(1-2):115-9 (1996); Amara, R.R., et al., Critical role for Env as well as Gag-Pol in control of a simian-human immunodeficiency virus 89.6P challenge by a DNA prime/recombinant modified vaccinia virus Ankara vaccine, J Virol. Jun;76(12):6138-46 (2002)).

Monomeric gp120 protein-derived from the SF2 lab strain provided neutralization of HIV-1 lab strains and protection against virus challenges in primate models (Verschoor, E.J., et al., (1999), "Comparison of immunity generated by nucleic acid, MF59 and ISCOM-formulated HIV-1 gp120 vaccines in rhesus macaques," J. Virology 73: 3292-3300). Primary gp120 protein derived from Thai E field strains provided cross-subtype neutralization of lab strains (VanCott et al. (1999) J. Virol 73: 4640-4650). Primary sub-type B oligomeric o-gp140 protein provided partial neutralization of subtype B primary (field) isolates (Barnett et al. (2001) J. Viol. 75:5526-5540). Primary sub-type B o-gp140 delV2 DNA prime plus protein boost provided potent neutralization of diverse subtype B primary isolates and protection against virus challenge in primate models (Cherpelis et al., (2000) J. Virol. 75:1547-1550).

Vaccine strategies for induction of potent, broadly reactive, neutralizing antibodies may be assisted by construction of Envelope polypeptide structures that expose conserved neutralizing epitopes, for example, variable-region modifications/deletions and de-glycosylations, envelope protein-receptor complexes, rational design based on crystal structure (e.g., beta-sheet deletions), and gp41-fusion domain based immunogens.

Stable CHO cell lines for envelope protein production have been developed using optimized envelope polypeptide coding sequences, including, but not limited to, the following: gp120, o-gp140, gp120delV2, o-gp140delV2, gp120delV1V2, o-gp140delV1V2.

Exemplary envelope proteins, and coding sequences thereof, for use in the present invention include, but are not limited to, gp120, gp140, oligomeric gp140, and gp160, including mutated or modified forms thereof (e.g., deletion of the V2 loop, mutations in cleavage sites, or mutations in glycosylation sites). In one embodiment, HIV envelope polypeptides that have been modified to expose the region of their polypeptide that binds to the CCR5 receptor are useful in the practice of the present invention, as well as polynucleotide sequences encoding such polypeptides. From the perspective of humoral immunity, it is useful to generate an immune response that provides neutralization of primary isolates that utilize the CCR5 chemokine co-receptor, which is believed to be important for virus entry (Zhu, T., et al. (1993) Science 261:1179-1181; Fiore, J., et al. (1994) Virology; 204:297-303). These and other exemplary polynucleotide constructs (e.g., a variety of envelope protein coding sequences), methods of making the polynucleotide constructs, corresponding polypeptide products, and methods of making polypeptides useful for HIV immunization have been previously described, for example, in the following: PCT International Publication Nos.: WO/00/39302; WO/00/39304; WO/02/04493; WO/03/004657; WO/03/004620; and WO/03/020876; US Patent No. 6,602,705; and US Published Patent Application Nos. 20030143248 , and 20020146683.

Although described with reference to HIV subtypes B and C as exemplary subtypes, the compositions and methods of the present invention are applicable to a wide variety of HIV subtypes, serotypes, or strains and immunogenic polypeptides encoded thereby, including but not limited to the following: HIV-1 subtypes, A through K, N and O, the identified CRFs (circulating recombinant forms), and HIV-2 strains and its subtypes. See, e.g., Myers, et al., Los Alamos Database, Los Alamos National Laboratory, Los Alamos, New Mexico; Myers, et al., Human Retroviruses and Aids, 1990, Los Alamos, New Mexico: Los Alamos National Laboratory.

Further modifications of Env include, but are not limited to, generating polynucleotides that encode Env polypeptides having mutations and/or deletions therein. For instance, some or all of hypervariable regions, V1, V2, V3, V4 and/or V5 can be deleted or modified as described herein, particularly regions V1, V2, and V3. V1 and V2 regions may mask CCR5 co-receptor binding sites. (See, e.g., Moulard, et al. (2002) Proc. Nat'l Acad. Sci 14:9405-9416).
Accordingly, in certain embodiments, some or all of the variable loop regions are deleted, for example to expose potentially conserved neutralizing epitopes. Further, deglycosylation of N-linked sites are also potential targets for modification inasmuch as a high degree of glycosylation also serves to shield potential neutralizing epitopes on the surface of the protein. Additional optional modifications, used alone or in combination with variable region deletes and/or deglycosylation modification, include modifications (e.g., deletions) to the beta-sheet regions (e.g., as described in WO 00/39303), modifications of the leader sequence (e.g., addition of Kozak sequences and/or replacing the modified wild type leader with a native or sequence-modified tpa leader sequence) and/or modifications to protease cleavage sites (e.g., Chakrabarti, et al., (2002) J. Virol. 76(11):5357-5368 describing a gp140 Delta CFI containing deletions in the cleavage site, fusogenic domain of gp41, and spacing of heptad repeats 1 and 2 of gp41 that retained native antigenic conformational determinants as defined by binding to known monoclonal antibodies or CD4, oligomer formation, and virus neutralization in vitro).

Various combinations of these modifications can be employed to generate wild-type or synthetic polynucleotide sequences as described herein.

Modification of the Env polypeptide coding sequences may result in (1) improved expression relative to the wild-type coding sequences in a number of mammalian cell lines (as well as other types of cell lines, including, but not limited to, insect cells), and/or (2) improved presentation of neutralizing epitopes. Similar Env polypeptide coding sequences can be obtained, modified and tested for improved expression from a variety of isolates.

As noted above, prime-boost methods are preferably employed where one or more gene delivery vectors are delivered in a "priming" step and, subsequently, one or more second gene delivery vectors are delivered in a "boosting" step. In certain embodiments, priming and boosting with one or more gene delivery vectors described herein is followed by additional boosting with one or more polypeptide-containing compositions (*e.g.*, polypeptides comprising HIV antigens).

In any method involving co-administration, the various compositions can be delivered in any order. Thus, in embodiments including delivery of multiple different compositions or molecules, the nucleic acids need not be all delivered before the polypeptides. For example, the priming step may include delivery of one or more polypeptides and the boosting comprises delivery of one or more nucleic acids and/or one more polypeptides. Multiple polypeptide administrations can be followed by multiple nucleic acid administrations or polypeptide and nucleic acid administrations can be performed in any order. Thus, one or more or the gene deliver vectors described herein and one or more of the polypeptides described herein can be co-administered in any order and via any administration routes. Therefore, any combination of polynucleotides and polypeptides described herein can be used to elicit an immune reaction.

In addition, following prime-boost regimes (such as those of the present invention described herein) may be beneficial to help reduce viral load in infected subjects, as well as possibly slow or prevent progression of HIV-related disease (relative to untreated subjects).

### EXPERIMENTAL

Below are examples of specific embodiments for carrying out the present invention. The examples are offered for illustrative purposes only, and are not intended to limit the scope of the present invention in any way.

Efforts have been made to ensure accuracy with respect to numbers used (e.g., amounts, temperatures, etc.), but some experimental error and deviation should, of course, be allowed for.

### Example 1

### Generation of Synthetic Expression Cassettes

### A. Generating Synthetic Polynucleotides

The polynucleotide sequences used in the practice of the present invention are typically manipulated to maximize expression of their gene products in a desired host or host cell. Following here is some exemplary guidance concerning codon optimization and functional variants of HIV polypeptides. The order of the following steps may vary.

First, the HIV-1 codon usage pattern may be modified so that the resulting nucleic acid coding sequence is comparable to codon usage found in highly expressed human genes. The HIV codon usage reflects a high content of the nucleotides A or T of the codon-triplet. The effect of the HIV-1 codon usage is a high AT content in the DNA sequence that results in a high AU content in the RNA and in a decreased translation ability and instability of the mRNA. In comparison, highly expressed human codons prefer the nucleotides G or C. Wildtype polynucleotide sequences encoding polypeptides are typically modified to be comparable to codon usage found in highly expressed human genes.

Second, for some genes variants are created (e.g., mutated forms of the wild-type polypeptide). In the following table (Table 2) mutations affecting the activity of several HIV genes are disclosed.

**Table 2**

| **Gene** | **"Region"** | **Exemplary Mutations** |
|---|---|---|
| Pol | prot | **Att** = Reduced activity by attenuation of Protease (Thr26Ser) (e.g., Konvalinka et al., 1995, J Virol 69: 7180-86) **Ina** = Mutated Protease, nonfunctional enzyme (Asp25Ala)(e.g., Konvalinka et al., 1995, J Virol 69: 7180-86) |
| | RT | **YM =** Deletion of catalytic center (YMDD_AP; SEQ ID NO:7) (e.g., Biochemistry, 1995, 34, 5351, Patel et. al.) **WM** = Deletion of primer grip region (WMGY_PI; SEQ ID NO:8) (e.g., J Biol Chem, 272, 17, 11157, Palaniappan, et. al., 1997) |
| | RNase | no direct mutations, RnaseH is affected by "WM" mutation in RT |
| | Integrase | 1) Mutation of HHCC domain, Cys40Ala (e.g., Wiskerchen et. al., 1995, J Virol, 69: 376). 2.) Inactivation catalytic center, Asp64Ala, Asp116Ala, Glu152Ala (e.g., Wiskerchen et. al., 1995, J Virol, 69: 376). 3) Inactivation of minimal DNA binding domain (MDBD), deletion of Trp235(e.g., Ishikawa et. al., 1999, J Virol, 73: 4475). Constructs int.opt.mut.SF2 and int.opt.mut_C (South Africa TV1) both contain all these mutations (1, 2, and 3) |
| Env | | Mutations in cleavage site (e.g., Earl et al. (1990) PNAS USA 87:648-652; Earl et al. (1991) J. Virol. 65:31-41). Mutations in glycosylation site (*e.g.*, GM mutants, for example, change Q residue in V1 and/or V2 to N residue; may also be designated by residue altered in sequence) Deletions or modifications of the V1, V2, V3, V4 or V5 regions or combinations thereof. (See e.g., US 6602705) Deletions or modifications of the β-sheet regions. (See e.g., WO 00/39303) |
| Tat | | Mutants of Tat in transactivation domain (e.g., Caputo et al., 1996, Gene Ther. 3:235), e.g., cys22 mutant (Cys22Gly), cys37 mutant (Cys37Ser), and double mutants |
| Rev | | Mutations in Rev domains (e.g., Thomas et al., 1998, J Virol. 72:2935-44), e.g., mutation in RNA binding-nuclear localization ArgArg38,39AspLeu, mutations in activation domain LeuGlu78,79AspLeu = M10 |
| Nef | | Mutations of myristoylation signal and in oligomerization domain, for example: 1. Single point mutation myristoylation signal: Gly-to-Ala 2. Deletion of N-terminal first 18 (sub-type B, e.g., SF162) or 19 (sub-type C, e.g., South Africa clones) amino acids. (e.g., Peng and Robert-Guroff, 2001, Immunol Letters 78:195-200) Single point mutation oligomerization: (e.g., Liu et al., 2000, J Virol 74: 5310-19) Mutations affecting (1) infectivity (replication) of HIV-virions and/or (2) CD4 down regulation. (*e.g*., Lundquist et al. (2002) J Virol. 76(9):4625-33) |
| Vif | | Mutations of Vif: e.g., Simon et al., 1999, J Virol 73:2675-81 |
| Vpr | | Mutations of Vpr: e.g., Singh et al., 2000, J Virol 74: 10650-57 |
| Vpu | | Mutations of Vpu: e.g., Tiganos et al., 1998, Virology 251: 96-107 |

Exemplary polynucleotides comprising some of these mutations have been previously described ( see, e.g., PCT International Publication Nos.: WO/00/39302; WO/00/39303; WO/00/39304; WO/02/04493; WO/03/004657; WO/03/004620; and WO/03/020876). Reducing or eliminating the function of the associated gene products can be accomplished employing the teachings set forth in the above table, in view of the teachings of the present specification.

In one aspect, the present invention comprises *Env* coding sequences that include, but are not limited to, polynucleotide sequences encoding the following HIV-encoded polypeptides: gp160, gp140, and gp120 (see, e.g., U.S. Patent No. 5,792,459 for a description of the HIV-1_{SF2} ("SF2") Env polypeptide). The relationships between these polypeptides can be readily determined. The polypeptide gp160 includes the coding sequences for gp120 and gp41. The polypeptide gp41 is comprised of several domains including an oligomerization domain (OD) and a transmembrane spanning domain (TM). In the native envelope, the oligomerization domain is required for the non-covalent association of three gp41 polypeptides to form a trimeric structure: through non-covalent interactions with the gp41 trimer (and itself), the gp120 polypeptides are also organized in a trimeric structure. A cleavage site (or cleavage sites) exists approximately between the polypeptide sequences for gp120 and the polypeptide sequences corresponding to gp41. This cleavage site(s) can be mutated to prevent cleavage at the site. The resulting gp140 polypeptide corresponds to a truncated form of gp160 where the transmembrane spanning domain of gp41 has been deleted. This gp140 polypeptide can exist in both monomeric and oligomeric (*i.e.* trimeric) forms by virtue of the presence of the oligomerization domain in the gp41 moiety. In the situation where the cleavage site has been mutated to prevent cleavage and the transmembrane portion of gp41 has been deleted the resulting polypeptide product is designated "mutated" gp140 (e.g., gp140.mut). As will be apparent to those in the field, the cleavage site can be mutated in a variety of ways. (See, also, e.g., PCT International Publication Nos. WO 00/39302 and WO/02/04493).

Wild-type HIV coding sequences (*e.g*., Gag, Env, Pol, tat, rev, nef, vpr, vpu, vif, etc.) can be selected from any known HIV isolate and these sequences manipulated to maximize expression of their gene products following the teachings of the present invention. The wild-type coding region maybe modified in one or more of the following ways: sequences encoding hypervariable regions of Env, particularly V1 and/or V2 are deleted, and/or mutations are introduced into sequences, for example, encoding the cleavage site in Env to abrogate the enzymatic cleavage of oligomeric gp140 into gp120 monomers. (See, e.g., Earl et al. (1990) PNAS USA 87:648-652; Earl et al. (1991) J. Virol. 65:31-41). In yet other embodiments, hypervariable region(s) are deleted, N-glycosylation sites are removed and/or cleavage sites mutated. As discussed above, different mutations may be introduced into the coding sequences of different genes (see, e.g., Table 2).

To create the synthetic coding sequences of the present invention the gene cassettes are designed to comprise the entire coding sequence of interest. Synthetic gene cassettes are constructed by oligonucleotide synthesis and PCR amplification to generate gene fragments. Primers are chosen to provide convenient restriction sites for subcloning. The resulting fragments are then ligated to create the entire desired sequence which is then cloned into an appropriate vector. The final synthetic sequences are (i) screened by restriction endonuclease digestion and analysis,(ii) subjected to DNA sequencing in order to confirm that the desired sequence has been obtained and (iii) the identity and integrity of the expressed protein confirmed by SDS-PAGE and Western blotting. The synthetic coding sequences are assembled at Chiron Corp. (Emeryville, CA) or by the Midland Certified Reagent Company (Midland, Texas).

Percent identity to the synthetic sequences of the present invention can be determined, for example, using the Smith-Waterman search algorithm (Time Logic, Incline Village, NV), with the following exemplary parameters: weight matrix = nuc4x4hb; gap opening penalty = 20, gap extension penalty = 5, reporting threshold = 1; alignment threshold = 20.

Various forms of the different embodiments of the present invention (*e.g.*, constructs) may be combined.

### Example 2

### Methods of Measuring Immune Response

### A Humoral Immune Response

The humoral immune response is checked with a suitable anti-HIV antibody ELISAs (enzyme-linked immunosorbent assays) of the mice sera 0 and 2-4 week intervals post immunization.

The antibody titers of the sera are determined by anti-HIV antibody ELISA. Briefly, sera from immunized mice are screened for antibodies directed against HIV envelope protein. ELISA microtiter plates are coated with 0.2 µg of HIV envelope gp140 protein per well overnight and washed four times; subsequently, blocking is done with PBS-0.2% Tween (Sigma) for 2 hours. After removal of the blocking solution, 100 µl of diluted mouse serum is added. Sera are tested at 1/25 dilutions and by serial 3-fold dilutions, thereafter. Microtiter plates are washed four times and incubated with a secondary, peroxidase-coupled anti-mouse IgG antibody (Pierce, Rockford, IL). ELISA plates are washed and 100 µl of 3, 3', 5, 5'-tetramethyl benzidine (TMB; Pierce) is added per well. The optical density of each well is measured after 15 minutes. The titers reported are the reciprocal of the dilution of serum that gave a half-maximum optical density (O.D.).

Ad5 and Ad7 microtiter neutralization assays were performed essentially as previously described in Buge, et al., J. Virol. 71:8531-8541 (1997) and Lubeck, et al., Nature Med. 3:651-8 (1997).

The results of these assays are used to show the potency of the polynucleotide/polypeptide immunization methods of the present invention for the generation of an immune response in mice.

### B. Cellular Immune Response

The frequency of specific cytotoxic T-lymphocytes (CTL) is evaluated by a standard chromium release assay of peptide pulsed Balb/c mouse CD4 cells. HIV protein-expressing vaccinia virus infected CD-8 cells may be used as a positive control (vv-protein). Briefly, spleen cells (Effector cells, E) are obtained from the BALB/c mice (immunized as described above). The cells are cultured, restimulated, and assayed for CTL activity against, e.g., Envelope peptide-pulsed target cells (see, e.g., Doe, B., and Walker, C.M., AIDS 10(7):793-794, 1996, for a general description of the assay). Cytotoxic activity is measured in a standard ⁵¹Cr release assay. Target (T) cells are cultured with effector (E) cells at various E:T ratios for 4 hours and the average cpm from duplicate wells is used to calculate percent specific ⁵¹Cr release. Antigen specific T cell responses in immunized mice can also be measured by flow cytometric determinations of intracellular cytokine production (Cytokine flow Cytometry or "CFC") as described by zur Megede, J., et al." in Expression and immunogenicity of sequence-modified human immunodeficiency virus type 1 subtype B pol and gagpol DNA vaccines, J Virol. 77:6197-207 (2003).

Cytotoxic T-cell (CTL) or CFC activity is measured in splenocytes recovered from the mice immunized with HIV DNA constructs and polypeptides as described herein. Effector cells from the immunized animals typically exhibit specific lysis of HIV peptide-pulsed SV-BALB (MHC matched) targets cells indicative of a CTL response. Target cells that are peptide-pulsed and derived from an MHC-unmatched mouse strain (MC57) are not lysed. The results of the CTL or CFC assays are used to show the potency of the polynucleotide/polypeptide immunization methods of the present invention for induction of cytotoxic T-lymphocyte (CTL) responses by DNA immunization.

### C. Generation of ADCC activity

As stated previously, antibody dependent cell cytotoxicity (ADCC) can also provide protection to an immunized host. Such responses can be determined using a variety of standard immunoassays that are well known in the art. (See, *e.g*., Montefiori et al. (1988) J. Clin Microbiol. 26:231-235; Dreyer et al. (1999) AIDS Res Hum Retroviruses (1999) 15(17):1563-1571).

### Example 3

### In vivo Immunogenicity Studies

### A. General Immunization Methods

To evaluate the immune response generated using the compositions (comprising a polynucleotide component and a polypeptide component) and methods of the present invention, studies using guinea pigs, rabbits, mice, rhesus macaques, baboons and/or chimpanzees may be performed. The studies are typically structured as shown in the following table (Table 3).

Preferably, animals are selected with minimal Ad5- and Ad7-cross-reactive antibodies.

The delAd5-E3, Ad7delE3, Ad5delE1/E3, and Ad7delE1/E3 vectors have been previously described (Nan X., et al., Development of an Ad7 cosmid system and generation of an Ad7deltaE1deltaE3HIV(MN) env/rev recombinant virus. (Gene Ther. 10(4):326-36 (2003)). Similarly, nonreplicating alphavirus vectors are described, for example, in Dubensky et al., J. Virol. (1996) 70:508-519; and International Publication Nos. WO 95/07995 and WO 96/17072; U.S. Patent No. 5,843,723; U.S. Patent No. 5,789,245; U.S. Patent No. 6,015,686; U.S. Patent No. 5,814,482; U.S. Patent No. 6,015,694, U.S. Patent No. 5,789,245, EP 1029068A2, International Publication No. WO 9918226; EP 00907746; International Publication No. WO 9738087A2, and Perriet al. (2003) J. Virol 77(19):10394-403.

**Table 3**

| **Priming phase** | **Boosting Phase 1** | **Boosting Phase 2** |
|---|---|---|
| Replicating Adenovirus | Non-replicating Alphavirus | None or adjuvant alone |
| Replicating Adenovirus | Non-replicating Alphavirus | protein Env + adjuvant |
| Non-replicating Adenovirus | Non-replicating Alphavirus | None or adjuvant alone |
| Non-replicating Adenovirus | Non-replicating Alphavirus | protein Env + adjuvant |
| Non-replicating Alphavirus | Non-replicating Ad | None or adjuvant alone |
| Non-replicating Alphavirus | Non-replicating Ad | protein Env + adjuvant |

The priming and boosting phases may use single or multiple administrations of vector or protein. The priming and boosting gene delivery vectors can encode analogous proteins from different subtypes, strains or isolates (*e*.*g*., *Env, Gag, Gagpol, rev* proteins from subtype B and subtype C). In a preferred embodiment, the polypeptide encoded is an env polypeptide. The optional protein(s) may be from one or more of the subtypes of the proteins encoded by the vectors or from one or more different subtypes. For example, the priming gene delivery vector may encode env from strain MN and the analogous boosting gene delivery vector may comprise env from SF162. As discussed further herein, the polypeptide and/or polynucleotide encoding the polypeptide may be truncated modified or otherwise altered to enhance immunogencity.

The amount of each DNA and /or protein in the mixed samples (i.e, B & C, in this example) can be added at an amount equal to that delivered in the single immunizations (such that 2X the amount of total DNA and/or protein is delivered) or the amount of each DNA and/or protein in the mixed samples can be adjusted so that the same total amount (1X) of DNA and/or protein is delivered in the mixed and single samples.

In addition to examples in Table 3 exemplifying combinations of polynucleotide component and polypeptide component, other combinations can be mentioned.

Any adjuvant can be used, for example, MF59C adjuvant, which is a microfluidized emulsion containing 5% squalene, 0.5% Tween 80, 0.5% Span 85, in 10mM citrate pH 6, stored in 10 ml aliquots at 4°C or the Iscomatrix adjuvant, which is a quil saporin based adjuvant used for protein delivery (available from, e.g., CSL Limited, Victoria, Australia).

### B. Mice

Experiments may be performed in mice following the immunization protocol illustrated in Table 3 and using the methods essentially as described in Example 2.

### C. Guinea Pigs

Experiments may be performed in guinea pigs as follows. Groups comprising six guinea pigs each are immunized parenterally (e.g., intramuscularly or intradermally) or mucosally at 0, 4, and 12 weeks with priming gene delivery vectors comprising expression cassettes comprising one or more HIV immunogenic polypeptide as illustrated in Table 3. A subset of the animals are subsequently boosted at approximately 12-24 weeks with a single dose (intramuscular, intradermally or mucosally) of the boosting gene delivery vector and, optionally, protein, as illustrated in Table 3. Animals may be boosted subsequently multiple times at 8-16 week intervals with the second gene delivery vector and, optionally, with HIV protein.

Antibody titers (geometric mean titers) are measured at two weeks following the third priming DNA immunization and at two weeks after the DNA boost. Results of these studies are used to demonstrate the usefulness of the compositions and methods of the invention to generate immune responses, in particular to generate broad and potent neutralizing activity against diverse HIV strains.

### D. Rabbits

Experiments may be performed in rabbits as follows. Rabbits are immunized intramuscularly or intradermally at multiple sites (using needle injection with or without subsequent electroporation, or using a Bioject needless syringe) or mucosally with priming gene delivery vectors comprising expression cassettes comprising one or more HIV immunogenic polypeptide. A subset of the animals are subsequently boosted with a single dose (intramuscular, intradermally or mucosally) of the boosting gene delivery vectors and, optionally, as illustrated in Table 3. Animals may be boosted multiple times with the boosting vector and optional protein.

Typically, the compositions of the present invention used to generate immune responses are highly immunogenic and generate substantial antigen binding antibody responses after only 2 immunizations in rabbits.

Results of these studies are used to demonstrate the usefulness of the compositions and methods of the invention to generate immune responses, in particular to generate broad and potent neutralizing activity against diverse HIV strains.

### E. Rhesus Macaques

Experiments may be performed in rhesus macaques as follows. Rhesus macaques are immunized at approximately 0, 4, 8, and 24 weeks parenterally or mucosally with priming gene delivery vectors comprising expression cassettes comprising one or more HIV immunogenic polypeptide as illustrated in Table 3. Enhanced DNA delivery systems such as use of DNA complexed to PLG microparticles or saline injection of DNA followed by electropoartion can be employed to increase immune response during the DNA priming phase of the immunization regimen.

A subset of the animals are subsequently boosted with a single dose (intramuscular, intradermally or mucosally) of the boosting gene delivery vector as illustrated in Table 3. Animals may be boosted multiple times generally at 3-6 month intervals with the boosting gene delivery vector and, optionally, HIV protein. Typically, the macaques have detectable HIV-specific T-cell responses as measured by CTL assays or Cytokine Flow Cytometry after two or three 1 mg doses of the polynucleotide component. Neutralizing antibodies may also detected. Results of these studies are used to demonstrate the usefulness of the compositions and methods of the invention to generate immune responses, in particular to generate broad and potent neutralizing activity against diverse HIV strains.

### F. Baboons

Baboons are immunized 4 times (at approximately weeks 0, 4, 8, and 24) intramuscular, or intradermally, or mucosally with priming gene delivery vectors comprising expression cassettes comprising one or more HIV immunogenic polypeptide as illustrated in Table 3. The priming gene delivery vector can be delivered in saline with or without electroporation, or on PLG microparticles. A subset of the animals are subsequently boosted with a single dose (intramuscular, intradermally or mucosally) of the boosting gene delivery vector and, optionally, HIV protein(s) as illustrated in Table 3. Animals may be boosted multiple times generally at 3-6 month intervals with the boosting vector and optional protein.

The animals are bled two-four weeks after each immunization and an HIV antibody ELISA is performed with isolated plasma. The ELISA is performed essentially as described below in Section G except the second antibody-conjugate is typically an anti-human IgG, g-chain specific, peroxidase conjugate (Sigma Chemical Co., St. Louis, MD 63178) used at a dilution of 1:500. Fifty µg/ml yeast extract may be added to the dilutions of plasma samples and antibody conjugate to reduce non-specific background due to preexisting yeast antibodies in the baboons. Lymphoproliferative responses to are typically observed in baboons post-boosting with HIV-polypeptide. Such proliferation results are indicative of induction of T-helper cell functions. Results of these studies are used to demonstrate the usefulness of the compositions and methods of the invention to generate immune responses, in particular to generate broad and potent neutralizing activity against diverse HIV strains.

### G. Humoral Immune Response

In any immunized animal model (including the above, as well as, for example, chimpanzees), the humoral immune response is checked in serum specimens from the immunized animals with an anti-HIV antibody ELISAs (enzyme-linked immunosorbent assays) at various times post-immunization as described in Example 2. Briefly, sera from immunized animals are screened for antibodies directed against the HIV polypeptide/protein(s) encoded by the DNA and/or polypeptide used to immunize the animals (e.g., oligomeric gp140). Typically independent ELISA assays are carried out using polypeptides corresponding to each of the subtypes used in the immunization study.

Wells of ELISA microtiter plates are coated overnight with the selected HIV polypeptide/protein and washed four times; subsequently, blocking is done with PBS-0.2% Tween (Sigma) for 2 hours. After removal of the blocking solution, 100 µl of diluted mouse serum is added. Sera are tested at 1/25 dilutions and by serial 3-fold dilutions, thereafter. Microtiter plates are washed four times and incubated with a secondary, peroxidase-coupled anti-mouse IgG antibody (Pierce, Rockford, IL). ELISA plates are washed and 100 µl of 3, 3', 5, 5'-tetramethyl benzidine (TMB; Pierce) was added per well. The optical density of each well is measured after 15 minutes. Titers are typically reported as the reciprocal of the dilution of serum that gave a half-maximum optical density (O.D.).

Cellular immune responses may also be evaluated as described in Example 2.

The presence of neutralizing antibodies in the sera is determined essentially as follows: Virus neutralization is measured in 5.25.EGFP.Luc.M7 (M7-luc) cells obtained from Dr. Nathaniel Landau (Salk Institute, San Diego, CA). The format of this assay is essentially the same as the MT-2 assay as described elsewhere (Montefiori et al. (1988) J. Clin Microbiol. 26:231-235) except that virus infection is quantified by luciferase reporter gene expression using a commercial luciferase kit (Promega). All serum samples are heat-inactivated for 1 hour at 56°C prior to assay. The virus stocks of the HIV-1 isolates are typically generated in PBMC.

Although preferred embodiments of the subject invention have been described in some detail, it is understood that obvious variations can be made without departing from the spirit and the scope of the invention. The following embodiments are offered for illustrative purposes only, and are not intended to limit the scope of the present invention in any way.

### Numbered embodiments of the invention

1. A composition for generating an immune response in a subject, the composition comprising,
   a first polynucleotide component encoding an HIV immunogenic polypeptide derived from a first HIV strain, and
   a second polynucleotide component encoding an HIV immunogenic polypeptide identical or analogous to the polypeptide encoded by the first polynucleotide component,
   wherein the first and second polynucleotide components comprise a gene delivery vector selected from the group consisting of a replicating adenoviral gene delivery vector and a non-replicating adenoviral or alphavirus gene delivery vector.
2. The composition of embodiment 1, wherein the second HIV strain is an HIV strain of the same subtype as the first HIV strain.
3. The composition of embodiment 1, wherein the second HIV strain is an HIV strain of a different subtype than the first HIV strain.
4. The composition of any of embodiments 1 to 3, further comprising a polypeptide component comprising one or more HIV immunogenic polypeptides.
5. The composition of embodiment 4, wherein one or more of the HIV immunogenic polypeptides are identical or analogous to the polypeptide encoded by the first or second polynucleotide component.
6. The composition of embodiment 5, wherein said the at least two of the HIV immunogenic polypeptides are derived from different HIV strains of different subtypes.
7. The composition of any of embodiments 1 to 6, wherein the first or second polynucleotide component or the polypeptide component comprises at least one native polynucleotide or polypeptide.
8. The composition of any of embodiments 1 to 6, wherein the first or second polynucleotide component comprises at least one synthetic polynucleotide.
9. The composition of embodiment 8, wherein the synthetic polynucleotide comprises codons altered for expression in mammalian cells.
10. The composition of embodiment 9, wherein the mammalian cells are human cells.
11. The composition of any of embodiments 1 to 10, wherein the first and second polynucleotide components encode polypeptides selected from the group consisting of one or more native HIV envelope polypeptides, one or more HIV Env polypeptides having an alteration or a mutation as compared to a native Env polypeptide and combinations thereof.
12. The composition of embodiment 11, wherein the alteration or mutation is selected from the group consisting of a mutation in the cleavage site, a mutation in the glycosylation site, a deletion or modification of the V1 region, a deletion or modification of the V2 region, a deletion or modification of the V3 region and combinations thereof.
13. The composition of embodiment 12, which exposes a neutralizing epitope of an HIV Env protein.
14. The composition of embodiment 13, wherein the neutralizing epitope comprises a CD4 binding region or an envelope binding region that binds to a CCR5 chemokine coreceptor.
15. The composition of any of embodiments 1 to 14, wherein the first HIV subtype is selected from the group consisting of: subtype A, subtype B, subtype C, subtype D, subtype E, subtype F, subtype G, subtype H, subtype I, subtype J, subtype K, subtype N and subtype O.
16. The composition of any of embodiments 1 to 15, wherein the polynucleotide components further comprise sequences encoding one or more control elements compatible with expression in a selected host cell, wherein the control elements are operable linked to polynucleotides encoding HIV immunogenic polypeptides.
17. The composition of embodiment 16, wherein the control elements are selected from the group consisting of a transcription promoter, a transcription enhancer element, a transcription termination signal, polyadenylation sequences, sequences for optimization of initiation of translation, an internal ribosome entry site, and translation termination sequences.
18. The composition of embodiment 17, wherein the transcription promoter is selected from the group consisting of CMV, CMV+intron A, SV40, RSV, HIV-Ltr, MMLV-ltr, and metallothionein.
19. The composition of any of embodiments 1 to 22, wherein at least one of the gene delivery vectors further comprises a carrier.
20. The composition of embodiment 19, wherein the carrier is selected from the group consisting of comprises a particulate carrier, a gold or tungsten particle, a PLG particle, and combinations thereof.
21. The composition of any of embodiments 1 to 20, wherein at least one of the gene delivery vectors is encapsulated in a liposome preparation.
22. The composition of any of embodiments 1 to 21, further comprising one or more additional gene delivery vectors selected from the group consisting of viral vectors, bacterial vectors and fungal vectors.
23. The composition of embodiment 22, wherein the viral vector is selected from the group consisting of different subtypes, species or serotypes of viral vectors.
24. The composition of embodiment 22 or 23, wherein the viral vector is selected from the group consisting of a retroviral vector, a lentiviral vector, an alphaviral vector, an adenoviral vector and combinations thereof.
25. The composition of embodiment 24, wherein the adenoviral vector is a live replicating vector or a non-replicating vector.
26. A method of generating an immune response in a subject, comprising, administering to the subject a composition according to any of embodiments 1 to 25.
27. The method of embodiment 26, wherein the first and second polynucleotide components of the composition are administered concurrently.
28. The method of embodiment 27, wherein the first and second polynucleotide components are administered sequentially.
29. The method of embodiment 26, 27 or 28, wherein the polypeptide component further comprises an adjuvant.
30. The method of any of embodiments 26 to 29, wherein the subject is a mammal.
31. The method of embodiment 30, wherein the mammal is a human.
32. The method of any of embodiments 26 to 32, wherein the immune response comprises a response selected from the group consisting of an adaptive immune response; an innate immune response; a humoral immune response; a cellular immune response and combinations thereof.
33. The method of embodiment 32, wherein the immune response comprises an Antibody Dependent Cell Mediated Cytotoxic (ADCC) response.
34. The method of embodiment 33, wherein the antibodies demonstrate ADCC activity against two or more HIV strains from two or more different HIV subtypes.
35. The method of embodiment 34, wherein the antibodies demonstrate ADCC activity against two or more HIV subtypes selected from the group consisting of the following HIV subtypes: A, B, C, D, E, F, G, and O.
36. The method of 32, wherein the immune response is a humoral immune response comprising the generation of neutralizing antibodies in the subject, wherein the neutralizing antibodies are selected from the group consisting of neutralizing antibodies against multiple strains derived from the first HIV subtype, neutralizing antibodies against multiple strains derived from the more than one HIV subtype, neutralizing antibodies that neutralize multiple HIV isolates, neutralizing antibodies that neutralize activity of two or more HIV strains from the same HIV subtype, neutralizing antibodies that neutralize activity of two or more HIV strains from two or more different HIV subtypes and combinations thereof.
37. The method of embodiment 36, wherein the broadly neutralizing antibodies neutralize activity of HIV strains utilizing the CCR5 co-receptor.
38. The method of any of embodiments 26 to 37, wherein at least one of the gene delivery vectors are administered intramuscularly, intramucosally, intranasally, subcutaneously, intradermally, transdermally, intravaginally, intrarectally, orally or intravenously.
39. The method as in any of embodiments 26-38, further comprising administering to the subject a polypeptide component comprising one or more HIV immunogenic polypeptides identical or analogous to the polypeptide encoded by the polynucleotide components.

## Claims

1. A composition comprising,
a first polynucleotide component encoding an HIV immunogenic polypeptide, wherein the HIV immunogenic polypeptide is from a first HIV, and
a second polynucleotide component encoding an HIV immunogenic polypeptide , wherein the HIV immunogenic polypeptide is from a second HIV strain,
wherein the first and second polynucleotide components comprise a gene delivery vector selected from the group consisting of a replicating adenoviral gene delivery vector and a non-replicating adenoviral or alphavirus gene delivery vector for use in generating an immune response in a patient wherein the first and second polynucleotide components are administered sequentially.

2. The composition for use in generating an immune response of claim 1, wherein the priming gene delivery vector is a replicating adenoviral gene delivery vector or a nonreplicating adenoviral gene delivery vector.

3. The composition for use in generating an immune response of claim 1 or claim 2, wherein the boosting gene delivery vector is an alphavirus gene delivery vector.

4. The composition for use in generating an immune response of claims 1-3, wherein the second HIV strain is an HIV strain of the same subtype or a different subtype as the first HIV strain.

5. The composition for use in generating an immune response of claims 1 to 4, further comprising a polypeptide component comprising one or more HIV immunogenic polypeptides.

6. The composition for use in generating an immune response of claim 5, wherein one or more of the HIV immunogenic polypeptides are from a third HIV strain.

7. The composition for use in generating an immune response of claims 1 to 6, wherein the first or second polynucleotide component comprises at least one synthetic polynucleotide.

8. The composition for use in generating an immune response of claim 7, wherein the synthetic polynucleotide comprises codons altered for expression in mammalian cells, such as human cells.

9. The composition for use in generating an immune response of claims 1 to 8, wherein the first and second polynucleotide components encode polypeptides selected from the group consisting of one or more HIV envelope polypeptides, one or more HIV Env polypeptides having an alteration or a mutation as compared to a native Env polypeptide and combinations thereof.

10. The composition for use in generating an immune response of claim 9, wherein the alteration or mutation is selected from the group consisting of a mutation in the cleavage site, a mutation in the glycosylation site, a deletion or modification of the V1 region, a deletion or modification of the V2 region, a deletion or modification of the V3 region and combinations thereof.

11. The composition for use in generating an immune response of claim 10, wherein the HIV Env protein comprises an epitope

12. The composition for use in generating an immune response of claims 1 to 11, wherein the first HIV subtype or group is selected from the group consisting of: subtype A, subtype B, subtype C, subtype D, subtype E, subtype F, subtype G, subtype H, subtype I, subtype J, subtype K, group N and group O.

13. The composition for use in generating an immune response of claims 1 to 15, wherein the polynucleotide components further comprise sequences encoding one or more control elements compatible with expression in a selected host cell, such as a transcription promoter, a transcription enhancer element, a transcription termination signal, polyadenylation sequences, sequences for optimization of initiation of translation, an internal ribosome entry site, or translation termination sequences, wherein the control elements are operable linked to polynucleotides encoding HIV immunogenic polypeptides.

14. The composition for use in generating an immune response of claim 1, wherein the polypeptide component further comprises an adjuvant.

15. The composition for use in generating an immune response of claims 1 to 14, wherein at least one of the gene delivery vectors are administered intramuscularly, intramucosally, intranasally, subcutaneously, intradermally, transdermally, intravaginally, intrarectally, orally or intravenously.
